# EUROPEAN PATENT SPECIFICATION

(11) **EP 1 051 620 B1**
(45) Date of publication and mention of the grant of the patent: **07.06.2006**
(21) Application number: 99971513.9
(22) Date of filing: 29.10.1999
(51) Int. Cl.: G01N 33/53, A61K 38/02, A61K 39/395, C07K 14/435, C07K 16/28, C12N 15/63, C12N 15/66, C12N 15/85, C12N 15/86, C12N 15/11

(54) **VARIABLE HEAVY CHAIN AND VARIABLE LIGHT CHAIN REGIONS OF ANTIBODIES TO HUMAN PLATELET GLYCOPROTEIN IB ALPHA**
REGIONEN VARIABLER SCHWERER KETTEN UND VARIABLER LEICHTER KETTEN VON ANTIKÖRPERN FÜR HUMANES BLUTPLÄTTCHEN-GLYKOPROTEIN IB ALPHA
REGIONS VARIABLES DE CHAINE LOURDE ET DE CHAINE LEGERE D'ANTICORPS DIRIGES CONTRE LA GLYCOPROTEINE PLAQUETTAIRE HUMAINE IB ALPHA

(30) Priority: 30.10.1998 US 106275 P
(43) Date of publication of application: 15.11.2000
(73) Proprietor: Miller, Jonathan L., Syracuse, NY 13224 (US)
(72) Inventor: Miller, Jonathan L., Syracuse, NY 13224 (US)
(74) Representative: Miles, John Stephen
(86) International application number: PCT/US1999/025495
(87) International publication number: WO 2000/026667

(56) References cited:
- WO-A-97/18236
- US-A- 5 817 748
- WARE J ET AL: "EXPRESSION OF HUMAN PLATELET GLYCOPROTEIN IBALPHA IN TRANSGENIC MICE" JOURNAL OF BIOLOGICAL CHEMISTRY, AMERICAN SOCIETY OF BIOLOGICAL CHEMISTS, BALTIMORE, MD, US, vol. 268, no. 11, 15 April 1993 (1993-04-15), pages 8376-8382, XP001115059 ISSN: 0021-9258
- STRASSEL CATHERINE ET AL: "A novel missense mutation shows that GPIbbeta has a dual role in controlling the processing and stability of the platelet GPIb-IX adhesion receptor." BIOCHEMISTRY. UNITED STATES 22 APR 2003, vol. 42, no. 15, 22 April 2003 (2003-04-22), pages 4452-4462, XP001094895 ISSN: 0006-2960
- GRIFFITHS ET AL.: 'Isolation of high affinity human antibodies directly from large synthetic repertoires' EMBO J., vol. 13, no. 14, 1994, pages 3245 - 3260, XP002926489
- NISSIM ET AL.: 'Antibodiy fragments from a 'single pot' phage display library as immunochemical reagents' EMBO J., vol. 13, no. 3, 1994, pages 692 - 698, XP002926488
- MILLER ET AL.: 'Mimotope/anti-mimotope probing of structural relationships in platelet glycoprotein Ibalpha' PROC. NATL. ACAD. SCI. USA, vol. 93, April 1996, pages 3565 - 3569, XP002926487
- KONKLE ET AL.: 'Cytokine-enhanced Expression of Glycoprotein Ibalpha in Human Endothelium' THE JOURNAL OF BIOLOGICAL CHEMISTRY, vol. 265, no. 32, 15 November 1990, pages 19833 - 19838, XP002926486

### SEQUENCE LISTING

<110> Miller, Jonathan L
<120> Variable Heavy Chain and Variable Light Chain Regions of Antibodies to Human Platelet Glycoprotein Ib Alpha
<120> 027.00030
<140> PCT/US99/25495
   <141> 1999-10-29
<150> US 60/106,275
   <151> 1998-10-30
<160> 61
<170> Patentin Ver. 2.1
<210> 1
   <211> 10
   <212> PRT
   <213> Artificial Sequence
<220>
   <223> Description of Artificial Sequence:Mimotope peptide directed to human monoclonal antibody C-34
<400> 1
   Ala Trp Asn Trp Arg Tyr Arg Glu Tyr Val
    1               5                   10
<210> 2
   <211> 339
   <212> DNA
   <213> Homo sapiens
<400> 2
   gaggtgcagc tggtggagtc tgggggaggt gtggtacggc ctggggggtc cctgagactc 60
   tcctgcgcag cctctggatt cacctttgat gattatggca tgagctgggt ccgccaagct 120
   ccagggaagg ggctggagtg ggtctccggt attaattgga atggtggtag cacaggttat 180
   gcagactctg tgaagggccg attcaccatc tccagagaca acgccaagaa ctccctgtat 240
   ctgcaaatga acagcctgag agccgaggac acggccgtgt attactgtgc aagattgaag 300
   atgcctcatg cgtggggcca aggtaccctg gtcaccgtc                        339
<210> 3
   <211> 300
   <212> DNA
   <213> Homo sapiens
<400> 3
   gggtccctta gactctcctg tgcagcctct ggattcactt tcagtaacgc ctggatgagc 60
   tgggtccgcc aggctccagg gaaggggctg gagtgggttg gccgtattaa aagcaaaact 120
   gatggtggga caacagacta cgctgcaccc gtgaaaggca gattcaccat ctcaagagat 180
   gattcaaaaa acacgctgta tctgcaaatg aacagcctga aaaccgagga cacggccgtg 240
   tattactgtg caagaaatcc gaagttggtg aagtggggcc aaggtaccct ggtcaccgtc 300
<210> 4
   <211> 336
   <212> DNA
   <213> Homo sapiens
<400> 4
   gaggtgcagc tggtggagtc tgggggaggc ttggtacagc ctggggggtc cctgagactc 60
   tcctgtgcag cctctggatt cacctttagc agctatgcca tgagctgggt ccgccaggct 120
   ccagggaagg ggctggagtg ggtctcagct attagtggta gtggtggtag cacatactac 180
   gcagactccg tgaagggccg gttcaccatc tccagagaca attccaagaa cacgctgtat 240
   ctgcaaatga acagcctgag agccgaggac acggccgtgt attactgtgc atggaagtct 300
   ttgcttatgc tttggggcca aggtaccctg gtcacc                           336
<210> 5
   <211> 324
   <212> DNA
   <213> Homo sapiens
<400> 5
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagctcagg aaacacagct tccttgacca tcactggggc tcaggcggaa 240
   gatgaggctg actattactg taactcccgg gacagcagtg gtaaccacgt attcggcgga 300
   gggaccaagc tgaccgtcct aggt                                        324
<210> 6
   <211> 330
   <212> DNA
   <213> Homo sapiens
<400> 6
   cagtctgtgc tgactcagcc accctcagcg tctgggaccc ccgggcagag ggtcaccatc 60
   tcttgttctg gaagcagctc caacatcgga agtaattatg tatactggta ccagcagctc 120
   ccaggaacgg cccccaaact cctcatctat aggaataatc agcggccctc aggggtccct 180
   gaccgattct ctggctccaa gtctggcacc tcagcctccc tggccatcag tgggctccgg 240
   tccgaggatg aggctgatta ttactgtgca gcatgggatg acagcctgtt gagtgtattc 300
   ggcggaggga ccaagctgac cgtcctaggt                                  330
<210> 7
   <211> 324
   <212> DNA
   <213> Homo sapiens
<400> 7
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagctcagg aaacacagct tccttgacca tcactggggc tcaggcggaa 240
   gatgaggctg actattactg taactcccgg gacagcagtg gtaaccatgt attcggcgga 300
   gggaccaagc tgaccgtcct aggt                                        324
<210> 8
   <211> 342
   <212> DNA
   <213> Homo sapiens
<400> 8
   gatgttgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ccttttacgt tcggccaagg gaccaagctg gaaatcaaac gt                 342
<210> 9
   <211> 336
   <212> DNA
   <213> Homo sapiens
<400> 9
   gagattgtga tgacccagac tccactctcc ttgtctatca cccctggaga gcaggcctcc 60
   atgtcctgca ggtctagtca gagcctcctg catagtgatg gatacaccta tttgtattgg 120
   tttctgcaga aagccaggcc agtctccacg ctcctgatct gtgaagtttc caaccggttc 180
   tcaggagtgc cagataggtt cagtggcagc gggtcaggga cagatttcac actgaaaatc 240
   agccgggtgg aggctgagga tgttggagtt tattactgca tgcaagatgc acaagatccc 300
   acgttcggcc aagggaccaa gctggaaatc aaacgt                      336
<210> 10
   <211> 113
   <212> PRT
   <213> Homo sapiens
<400> 10
   Glu Val Gln Leu Val Glu Ser Gly Gly Gly Val Val Arg Pro Gly Gly
   1               5                   10                  15
   Ser Leu Arg Leu Ser Cys Ala Ala Ser Gly Phe Thr Phe Asp Asp Tyr
               20                  25                  30
   Gly Met Ser Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Leu Lys Met Pro His Ala Trp Gly Gln Gly Thr Leu Val Thr
               100                 105                 110
   Val
<210> 11
   <211> 113
   <212> PRT
   <213> Homo sapiens
<400> 11
   Glu Val Gln Leu Val Glu Ser Gly Gly Gly Val Val Arg Pro Gly Gly
   1               5                   10                  15
   Ser Leu Arg Leu Ser Cys Ala Ala Ser Gly Phe Thr Phe Asp Asp Tyr
               20                  25                  30
   Gly Met Ser Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Leu Tyr His Cys
   85 90 95
   Ala Arg Leu Lys Met Pro His Ala Trp Gly Gln Gly Thr Leu Val Thr
   100 105 110
   Val
<210> 12
   <211> 100
   <212> FRT
   <213> Homo sapiens
<400> 12
   Gly Ser Leu Arg Leu Ser Cys Ala Ala Ser Gly Phe Thr Phe Ser Asn
   1 5 10 15
   Ala Trp Met Ser Trp Val Arg Gln Ala Pro Gly Lys Gly Leu Glu Trp
   20 25 30
   Val Gly Arg Ile Lys Ser Lys Thr Asp Gly Gly Thr Thr Asp Tyr Ala
   35 40 45
   Ala Pro Val Lys Gly Arg Phe Thr Ile Ser Arg Asp Asp Ser Lys Asn
   50 55 60
   Thr Leu Tyr Leu Gln Met Asn Ser Leu Lys Thr Glu Asp Thr Ala Val
   65 70 75 80
   Tyr Tyr Cys Ala Arg Asn Pro Lys Leu Val Lys Trp Gly Gln Gly Thr
   85 90 95
   Leu Val Thr Val
   100
<210> 13
   <211> 100
   <212> PRT
   <213> Homo sapiens
<400> 13
<210> 14
   <211> 112
   <212> PRT
   <213> Homo sapiens
<400> 14
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Trp Lys Ser Leu Leu Met Leu Trp Gly Gln Gly Thr Leu Val Thr
   100 105 110
<210> 15
   <211> 112
   <212> PRT
   <213> Homo sapiens
<400> 15
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Lys Ser Leu Leu Met Leu Trp Gly Gln Gly Thr Leu Val Thr
   100 105 110
<210> 16
   <211> 108
   <212> PRT
   <213> Homo sapiens
<400> 16
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Ser Ser Gly Asn His
   85 90 95
   Val Phe Gly Gly Gly Thr Lys Leu Thr Val Leu Gly
   100 105
<210> 17
   <211> 110
   <212> PRT
   <213> Homo sapiens
<400> 17
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ser Asn
   20 25 30
   Tyr Val Tyr Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Ser Leu
   85 90 95
   Leu Ser Val Phe Gly Gly Gly Thr Lys Leu Thr Val Leu Gly
   100 105 110
<210> 18
   <211> 108
   <212> PRT
   <213> Homo sapiens
<400> 18
   Ser Ser Glu Leu Thr Gln Asp Pro Ala Val Ser Val Ala Leu Gly Gln
    1               5                  10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Asn Ser Arg Asp Ser Ser Gly Asn His
                   85                  90                  95
   Val Phe Gly Gly Gly Thr Lys Leu Thr Val Leu Gly
               100                 105
<210> 19
   <211> 114
   <212> PRT
   <213> Homo sapiens
<400> 19
   Asp Val Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Pro Phe Thr Phe Gly Gln Gly Thr Lys Leu Glu Ile
   100 105 110
   Lys Arg
<210> 20
   <211> 112
   <212> PRT
   <213> Homo sapiens
<400> 20
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Pro Thr Phe Gly Gln Gly Thr Lys Leu Glu Ile Lys Arg
   100 105 110
<210> 21
   <211> 112
   <212> PRT
   <213> Homo sapiens
<400> 21
   Glu Ile Val Met Thr Gln Thr Pro Leu Ser Leu Ser Ile Thr Pro Gly
   1 5 10 15
   Glu Gln Ala Ser Met Ser Cys Arg Ser Ser Gln Ser Leu Leu His Ser
   20 25 30
   Asp Gly Tyr Thr Tyr Leu Tyr Trp Phe Leu Gln Lys Ala Arg Pro Val
   35 40 45
   Ser Thr Leu Leu Ile Cys Glu Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Asp
   85 90 95
   Ala Gln Asp Pro Thr Phe Gly Gln Gly Thr Lys Leu Glu Ile Lys Arg
   100 105 110
<210> 22
   <211> 238
   <212> PRT
   <213> Homo sapiens
<400> 22
   Glu Val Gln Leu Val Glu Ser Gly Gly Gly Val Val Arg Pro Gly Gly
   1 5 10 15
   Ser Leu Arg Leu Ser Cys Ala Ala Ser Gly Phe Thr Phe Asp Asp Tyr
   20 25 30
   Gly Met Ser Trp Val Arg Gln Ala Pro Gly Lys Gly Leu Glu Trp Val
   35 40 45
   Ser Gly Ile Asn Trp Asn Gly Gly Ser Thr Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Leu Lys Met Pro His Ala Trp Gly Gln Gly Thr Leu Val Thr
   100 105 110
   Val Ser Ser Gly Gly Gly Gly Ser Gly Gly Gly Gly Ser Gly Gly Ser
   115 120 125
   Ala Leu Ser Ser Glu Leu Thr Gln Asp Pro Ala Val Ser Val Ala Leu
   130 135 140
   Gly Gln Thr Val Arg Ile Thr Cys Gln Gly Asp Ser Leu Arg Ser Tyr
   145 150 155 160
   Tyr Ala Ser Trp Tyr Gln Gln Lys Pro Gly Gln Ala Pro Val Leu Val
   165 170 175
   Ile Tyr Gly Lys Asn Asn Arg Pro Ser Gly Ile Pro Asp Arg Phe Ser
   180 185 190
   Gly Ser Ser Ser Gly Asn Thr Ala Ser Leu Thr Ile Thr Gly Ala Gln
   195 200 205
   Ala Glu Asp Glu Ala Asp Tyr Tyr Cys Asn Ser Arg Asp Ser Ser Gly
   210 215 220
   Asn His Val Phe Gly Gly Gly Thr Lys Leu Thr Val Leu Gly
   225 230 235
<210> 23
   <211> 242
   <212> PRT
   <213> Homo sapiens
<400> 23
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Asn Ala
   20 25 30
   Trp Met Ser Trp Val Arg Gln Ala Pro Gly Lys Gly Leu Glu Trp Val
   35 40 45
   Gly Arg Ile Lys Ser Lys Thr Asp Gly Gly Thr Thr Asp Tyr Ala Ala
   50 55 60
   Pro Val Lys Gly Arg Phe Thr Ile Ser Arg Asp Asp Ser Lys Asn Thr
   65 70 75 80
   Leu Tyr Leu Gln Met Asn Ser Leu Lys Thr Glu Asp Thr Ala Val Tyr
   85 90 95
   Tyr Cys Ala Arg Asn Pro Lys Leu Val Lys Trp Gly Gln Gly Thr Leu
   100 105 110
   Val Thr Val Ser Ser Gly Gly Gly Gly Ser Gly Gly Gly Gly Ser Gly
   115 120 125
   Gly Ser Ala Leu Gln Ser Val Leu Thr Gln Pro Pro Ser Ala Ser Gly
   130 135 140
   Thr Pro Gly Gln Arg Val Thr Ile Ser Cys Ser Gly Ser Ser Ser Asn
   145 150 155 160
   Ile Gly Ser Asn Tyr Val Tyr Trp Tyr Gln Gln Leu Pro Gly Thr Ala
   165 170 175
   Pro Lys Leu Leu Ile Tyr Arg Asn Asn Gln Arg Pro Ser Gly Val Pro
   180 185 190
   Asp Arg Phe Ser Gly Ser Lys Ser Gly Thr Ser Ala Ser Leu Ala Ile
   195 200 205
   Ser Gly Leu Arg Ser Glu Asp Glu Ala Asp Tyr Tyr Cys Ala Ala Trp
   210 215 220
   Asp Asp Ser Leu Leu Ser Val Phe Gly Gly Gly Thr Lys Leu Thr Val
   225 230 235 240
   Leu Gly
<210> 24
   <211> 238
   <212> PRT
   <213> Homo sapiens
<400> 24
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Trp Lys Ser Leu Leu Met Leu Trp Gly Gln Gly Thr Leu Val Thr
   100 105 110
   Val Ser Ser Gly Gly Gly Gly Ser Gly Gly Gly Gly Ser Gly Gly Ser
   115 120 125
   Ala Leu Ser Ser Glu Leu Thr Gln Asp Pro Ala Val Ser Val Ala Leu
   130 135 140
   Gly Gln Thr Val Arg Ile Thr Cys Gln Gly Asp Ser Leu Arg Ser Tyr
   145 150 155 160
   Tyr Ala Ser Trp Tyr Gln Gln Lys Pro Gly Gln Ala Pro Val Leu Val
   165 170 175
   Ile Tyr Gly Lys Asn Asn Arg Pro Ser Gly Ile Pro Asp Arg Phe Ser
   180 185 190
   Gly Ser Ser Ser Gly Asn Thr Ala Ser Leu Thr Ile Thr Gly Ala Gln
   195 200 205
   Ala Glu Asp Glu Ala Asp Tyr Tyr Cys Asn Ser Arg Asp Ser Ser Gly
   210 215 220
   Asn His Val Phe Gly Gly Gly Thr Lys Leu Thr Val Leu Gly
   225 230 235
<210> 25
   <211> 131
   <212> PRT
   <213> Homo sapiens
<400> 25
   Ser Ser Gly Gly Gly Gly Ser Gly Gly Gly Gly Ser Gly Gly Ser Ala
   1               5                   10                  15
   Leu Asp Val Val Met Thr Gln Ser Pro Leu Ser Leu Pro Val Thr Pro
               20                  25                  30
   Gly Glu Pro Ala Ser Ile Ser Cys Arg Ser Ser Gln Ser Leu Leu His
           35                  40                  45
   Ser Asn Gly Tyr Asn Tyr Leu Asp Trp Tyr Leu Gln Lys Pro Gly Gln
       50                  55                  60
   Ser Pro Gln Leu Leu Ile Tyr Leu Gly Ser Asn Arg Ala Ser Gly Val
   65                  70                  75                  80
   Pro Asp Arg Phe Ser Gly Ser Gly Ser Gly Thr Asp Phe Thr Leu Lys
                   85                  90                  95
   Ile Ser Arg Val Glu Ala Glu Asp Val Gly Val Tyr Tyr Cys Met Gln
               100                 105                 110
   Ala Leu Gln Thr Pro Pro Phe Thr Phe Gly Gln Gly Thr Lys Leu Glu
           115                 120                 125
   Ile Lys Arg
       130
<210> 26
   <211> 129
   <212> PRT
   <213> Homo sapiens
<400> 26
<210> 27
   <211> 98
   <212> PRT
   <213> Homo sapiens
<400> 27
   Glu Val Gln Leu Val Glu Ser Gly Gly Gly Val Val Arg Pro Gly Gly
   1 5 10 15
   Ser Leu Arg Leu Ser Cys Ala Ala Ser Gly Phe Thr Phe Asp Asp Tyr
   20 25 30
   Gly Met Ser Trp Val Arg Gln Ala Pro Gly Lys Gly Leu Glu Trp Val
   35 40 45
   Ser Gly Ile Asn Trp Asn Gly Gly Ser Thr Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg
<210> 28
   <211> 98
   <212> PRT
   <213> Homo sapiens
<400> 28
   Glu Val Gln Leu Val Glu Ser Gly Gly Gly Val Val Arg Pro Gly Gly
   1 5 10 15
   Ser Leu Arg Leu Ser Cys Ala Ala Ser Gly Phe Thr Phe Asp Asp Tyr
   20 25 30
   Gly Met Ser Trp Val Arg Gln Ala Pro Gly Lys Gly Leu Glu Trp Val
   35 40 45
   Ser Gly Ile Asn Trp Asn Gly Gly Ser Thr Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Leu Tyr His Cys
   85 90 95
   Ala Arg
<210> 29
   <211> 5
   <212> PRT
   <213> Homo sapiens
<400> 29
   Asp Tyr Gly Met Ser
   1 5
<210> 30
   <211> 17
   <212> PRT
   <213> Homo sapiens
<400> 30
   Gly Ile Asn Trp Asn Gly Gly Ser Thr Gly Tyr Ala Asp Ser Val Lys
   1 5 10 15
   Gly
<210> 31
   <211> 100
   <212> PRT
   <213> Homo sapiens
<400> 31
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Asn Ala
   20 25 30
   Trp Met Ser Trp Val Arg Gln Ala Pro Gly Lys Gly Leu Glu Trp Val
   35 40 45
   Gly Arg Ile Lys Ser Lys Thr Asp Gly Gly Thr Thr Asp Tyr Ala Ala
   50 55 60
   Pro Val Lys Gly Arg Phe Thr Ile Ser Arg Asp Asp Ser Lys Asn Thr
   65 70 75 80
   Leu Tyr Leu Gln Met Asn Ser Leu Lys Thr Glu Asp Thr Ala Val Tyr
   85 90 95
   Tyr Cys Ala Arg
   100
<210> 32
   <211> 100
   <212> PRT
   <213> Homo sapiens
<400> 32
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Asn Ala
   20 25 30
   Trp Met Ser Trp Val Arg Gln Ala Pro Gly Lys Gly Leu Glu Trp Val
   35 40 45
   Gly Arg Ile Lys Ser Lys Thr Asp Gly Gly Thr Thr Asp Tyr Ala Ala
   50 55 60
   Pro Val Lys Gly Arg Phe Thr Ile Ser Arg Asp Asp Ser Lys Asn Thr
   65 70 75 80
   Leu Tyr Leu Gln Met Asn Ser Leu Lys Thr Glu Asp Thr Ala Val Tyr
   85 90 95
   Tyr Cys Thr Thr
   100
<210> 33
   <211> 5
   <212> PRT
   <213> Home sapiens
<400> 33
   Asn Ala Trp Met Ser
   1 5
<212> 34
   <211> 19
   <212> PRT
   <213> Homo sapiens
<400> 34
<210> 35
   <211> 98
   <212> PRT
   <213> Homo sapiens
<400> 35
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Trp
<210> 36
   <211> 98
   <212> PRT
   <213> Homo sapiens
<400> 36
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg
<210> 37
   <211> 5
   <212> PRT
   <213> Homo sapiens
<400> 37
   Ser Tyr Ala Met Ser
   1 5
<210> 38
   <211> 17
   <212> PRT
   <213> Homo sapiens
<400> 38
   Ala Ile Ser Gly Ser Gly Gly Ser Thr Tyr Tyr Ala Asp Ser Val Lys
   1 5 10 15
   Gly
<210> 39
   <211> 6
   <212> PRT
   <213> Homo sapiens
<400> 39
   Leu Lys Met Pro His Ala
   1 5
<210> 40
   <211> 6
   <212> PRT
   <213> Homo sapiens
<400> 40
   Asn Pro Lys Leu Val Lys
   1 5
<210> 41
   <211> 6
   <212> PRT
   <213> Homo sapiens
<400> 41
   Lys Ser Leu Leu Met Leu
   1 5
<210> 42
   <211> 96
   <212> PRT
   <213> Homo sapiens
<400> 42
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Ser Ser Gly Asn His
   85 90 95
<210> 43
   <211> 11
   <212> PRT
   <213> Homo sapiens
<400> 43
   Gln Gly Asp Ser Leu Arg Ser Tyr Tyr Ala Ser
   1 5 10
<210> 44
   <211> 7
   <212> PRT
   <213> Homo sapiens
<400> 44
   Gly Lys Asn Asn Arg Pro Ser
   1 5
<210> 45
   <211> 9
   <212> PRT
   <213> Homo sapiens
<400> 45
   Asn Ser Arg Asp Ser Ser Gly Asn His
   1 5
<210> 46
   <211> 98
<212> PRT
   <213> Homo sapiens
<400> 46
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ser Asn
   20 25 30
   Tyr Val Tyr Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Ser Leu
   85 90 95
   Leu Ser
<210> 47
   <211> 98
   <212> PRT
   <213> Homo sapiens
<400> 47
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ser Asn
   20 25 30
   Tyr Val Tyr Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Ser Leu
   85 90 95
   Ser Gly
<210> 43
   <211> 13
   <212> PRT
   <213 Homo sapiens
<400> 48
<210> 49
   <211> 7
   <212> PRT
   <213> Homo sapiens
<400> 49
   Arg Asn Asn Gln Arg Pro Ser
   1 5
<210> 50
   <211> 9
   <212> PRT
   <213> Homo sapiens
<400> 50
   Ala Ala Trp Asp Asp Ser Leu Leu Ser
   1 5
<210> 51
   <211> 96
   <212> PRT
   <213> Homo sapiens
<400> 51
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Ser Ser Gly Asn His
   85 90 95
<210> 52
   <211> 11
   <212> PRT
   <213> Homo sapiens
<400> 52
   Gln Gly Asp Ser Leu Arg Ser Tyr Tyr Ala Ser
   1 5 10
<210> 53
   <211> 7
   <212> PRT
   <213> Homo sapiens
<400> 53
   Gly Lys Asn Asn Arg Pro Ser
   1 5
<210> 54
   <211> 9
   <212> PRT
   <213> Homo sapiens
<400> 54
   Asn Ser Arg Asp Ser Ser Gly Asn His
   1 5
<210> 55
   <211> 16
   <212> PRT
   <213> Hemo sapiens
<400> 55
   Arg Ser Ser Gln Ser Leu Leu His Ser Asn Gly Tyr Asn Tyr Leu Asp
   1 5 10 15
<210> 56
   <211> 7
   <212> PRT
   <213> Homo sapiens
<400> 56
   Leu Gly Ser Asn Arg Ala Ser
   1 5
<210> 57
   <211> 9
   <212> PRT
   <213> Homo sapiens
<400> 57
   Met Gln Ala Leu Gln Thr Pro Pro Phe
   1 5
<210> 58
   <211> 7
   <212> PRT
   <213> Homo sapiens
<400> 58
   Met Gln Ala Leu Gln Thr Pro
   1 5
<210> 59
   <211> 102
   <212> PRT
   <213> Homo sapiens
<400> 59
   Asp Val Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Pro Pro Phe
   100
<210> 60
   <211> 100
   <212> PRT
   <213> Homo sapiens
<400> 60
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Pro
   100
<210> 61
   <211> 100
   <212> PRT
   <213> Homo sapiens
<400> 61
   Glu Ile Val Met Thr Gln Thr Pro Leu Ser Leu Ser Ile Thr Pro Gly
     1               5                  10                  15
   Glu Gln Ala Ser Met Ser Cys Arg Ser Ser Gln Ser Leu Leu His Ser
                20                  25                  30
   Asp Gly Tyr Thr Tyr Leu Tyr Trp Phe Leu Gln Lys Ala Arg Pro Val
            35                  40                  45
   Ser Thr Leu Leu Ile Cys Glu Val Ser Asn Arg Phe Ser Gly Val Pro
        50                  55                  60
   Asp Arg Phe Ser Gly Ser Gly Ser Gly Thr Asp Phe Thr Leu Lys Ile
    65                  70                  75                  80
   Ser Arg Val Glu Ala Glu Asp Val Gly Val Tyr Tyr Cys Met Gln Asp
                    85                  90                  95
   Ala Gln Asp Pro
               100

### SEQUENCE LISTING

<110> Miller, Jonathan L
<120> Variable Heavy Chain and Variable Light Chain Regions of Antibodies to Human Platelet Glycoprotein Ib Alpha
<130> 027.00030
<140> PCT/US99/25495
   <141> 1999-10-29
<150> US 60/106,275
   <151> 1998-10-30
<160> 61
<170> PatentIn Ver. 2.1
<210> 1
   <211> 10
   <212> PRT
   <213> Artificial Sequence
<220>
   <223> Description of Artificial Sequence:Mimotope peptide directed to human monoclonal antibody C-34
<400> 1
   Ala Trp Asn Trp Arg Tyr Arg Glu Tyr Val
   1 5 10
<210> 2
   <211> 339
   <212> DNA
   <313> Homo sapiens
<400> 2
   gaggtgcagc tggtggagtc tgggggaggt gtggtacggc ctggggggtc cctgagactc 60
   tcctgcgcag cctctggatt cacctttgat gattatggca tgagctgggt ccgccaagct 120
   ccagggaagg ggctggagtg ggtctccggt attaattgga atggtggtag cacaggttat 180
   gcagactctg tgaagggccg attcaccatc tccagagaca acgccaagaa ctccctgtat 240
   ctgcaaatga acagcctgag agccgaggac acggccgtgt attactgtgc aagattgaag 300
   atgcctcatg cgtggggcca aggtaccctg gtcaccgtc 339
<210> 3
   <211> 300
   <212> DNA
   <213> Homo sapiens
<400> 3
   gggtccctta gactctcctg tgcagcctct ggattcactt tcagtaacgc ctggatgagc 60
   tgggtccgcc aggctccagg gaaggggctg gagtgggttg gccgtattaa aagcaaaact 120
   gatggtggga caacagacta cgctgcaccc gtgaaaggca gattcaccat ctcaagagat 180
   gattcaaaaa acacgctgta tctgcaaatg aacagcctga aaaccgagga cacggccgtg 240
   tattactgtg caagaaatcc gaagttggtg aagtggggcc aaggtaccct ggtcaccgtc 300
<210> 4
   <211> 336
   <212> DNA
   <213> Homo sapiens
<400> 4
   gaggtgcagc tggtggagtc tgggggaggc ttggtacagc ctggggggtc cctgagactc 60
   tcctgtgcag cctctggatt cacctttagc agctatgcca tgagctgggt ccgccaggct 120
   ccagggaagg ggctggagtg ggtctcagct attagtggta gtggtggtag cacatactac 180
   gcagactccg tgaagggccg gttcaccatc tccagagaca attccaagaa cacgctgtat 240
   ctgcaaatga acagcctgag agccgaggac acggccgtgt attactgtgc atggaagtct 300
   ttgcttatgc tttggggcca aggtaccctg gtcacc 336
<210> 5
   <211> 324
   <212> DNA
   <213> Homo sapiens
<400> 5
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagctcagg aaacacagct tccttgacca tcactggggc tcaggcggaa 240
   gatgaggctg actattactg taactcccgg gacagcagtg gtaaccacgt attcggcgga 300
   gggaccaagc tgaccgtcct aggt 324
<210> 6
   <211> 330
   <212> DNA
   <213> Homo sapiens
<400> 6
   cagtctgtgc tgactcagcc accctcagcg tctgggaccc ccgggcagag ggtcaccatc 60
   tcttgttctg gaagcagctc caacatcgga agtaattatg tatactggta ccagcagctc 120
   ccaggaacgg cccccaaact cctcatctat aggaataatc agcggccctc aggggtccct 180
   gaccgattct ctggctccaa gtctggcacc tcagcctccc tggccatcag tgggctccgg 240
   tccgaggatg aggctgatta ttactgtgca gcatgggatg acagcctgtt gagtgtattc 300
   ggcggaggga ccaagctgac cgtcctaggt 330
<210> 7
   <211> 324
   <212> DNA
   <213> Homo sapiens
<400> 7
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagctcagg aaacacagct tccttgacca tcactggggc tcaggcggaa 240
   gatgaggctg actattactg taactcccgg gacagcagtg gtaaccatgt attcggcgga 300
   gggaccaagc tgaccgtcct aggt 324
<210> 8
   <211> 342
   <212> DNA
   <213> Homo sapiens
<400> 8
   gatgttgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ccttttacgt tcggccaagg gaccaagctg gaaatcaaac gt 342
<210> 9
   <211> 336
   <212> DNA
   <213> Homo sapiens
<400> 9
   gagattgtga tgacccagac tccactctcc ttgtctatca cccctggaga gcaggcctcc 60
   atgtcctgca ggtctagtca gagcctcctg catagtgatg gatacaccta tttgtattgg 120
   tttctgcaga aagccaggcc agtctccacg ctcctgatct gtgaagtttc caaccggttc 180
   tcaggagtgc cagataggtt cagtggcagc gggtcaggga cagatttcac actgaaaatc 240
   agccgggtgg aggctgagga tgttggagtt tattactgca tgcaagatgc acaagatccc 300
   acgttcggcc aagggaccaa gctggaaatc aaacgt 336
<210> 10
   <211> 113
   <212> PRT
   <213> Homo sapiens
<400> 10
   Glu Val Gln Leu Val Glu Ser Gly Gly Gly Val Val Arg Pro Gly Gly
    1               5                  10                  15
   Ser Leu Arg Leu Ser Cys Ala Ala Ser Gly Phe Thr Phe Asp Asp Tyr
               20                  25                  30
   Gly Met Ser Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Leu Lys Met Pro His Ala Trp Gly Gln Gly Thr Leu Val Thr
               100                 105                 110
   Val
<210> 11
   <211> 113
   <212> PRT
   <213> Homo sapiens
<400> 11
   Glu Val Gln Leu Val Glu Ser Gly Gly Gly Val Val Arg Pro Gly Gly
    1               5                  10                  15
   Ser Leu Arg Leu Ser Cys Ala Ala Ser Gly Phe Thr Phe Asp Asp Tyr
               20                  25                  30
   Gly Met Ser Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Leu Tyr His Cys
   85 90 95
   Ala Arg Leu Lys Met Pro His Ala Trp Gly Gln Gly Thr Leu Val Thr
   100 105 110
   Val
<210> 12
   <211> 100
   <212> PRT
   <213> Homo sapiens
<400> 12
   Gly Ser Leu Arg Leu Ser Cys Ala Ala Ser Gly Phe Thr Phe Ser Asn
   1 5 10 15
   Ala Trp Met Ser Trp Val Arg Gln Ala Pro Gly Lys Gly Leu Glu Trp
   20 25 30
   Val Gly Arg Ile Lys Ser Lys Thr Asp Gly Gly Thr Thr Asp Tyr Ala
   35 40 45
   Ala Pro Val Lys Gly Arg Phe Thr Ile Ser Arg Asp Asp Ser Lys Asn
   50 55 60
   Thr Leu Tyr Leu Gln Met Asn Ser Leu Lys Thr Glu Asp Thr Ala Val
   65 70 75 80
   Tyr Tyr Cys Ala Arg Asn Pro Lys Leu Val Lys Trp Gly Gln Gly Thr
   85 90 95
   Leu Val Thr Val
   100
<210> 13
   <211> 100
   <212> PRT
   <213> Homo sapiens
<210> 14
   <211> 112
   <212> PRT
   <213> Homo sapiens
<400> 14
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Trp Lys Ser Leu Leu Met Leu Trp Gly Gln Gly Thr Leu Val Thr
   100 105 110
<210> 15
   <211> 112
   <212> PRT
   <213> Homo sapiens
<400> 15
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Lys Ser Leu Leu Met Leu Trp Gly Gln Gly Thr Leu Val Thr
               100                 105                 110
<210> 16
   <211> 108
   <212> PRT
   <213> Homo sapiens
<400> 16
   Ser Ser Glu Leu Thr Gln Asp Pro Ala Val Ser Val Ala Leu Gly Gln
    1               5                  10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Ser Ser Gly Asn His
   85 90 95
   Val Phe Gly Gly Gly Thr Lys Leu Thr Val Leu Gly
   100 105
<210> 17
   <211> 110
   <212> PRT
   <213> Homo sapiens
<400> 17
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ser Asn
   20 25 30
   Tyr Val Tyr Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Ser Leu
   85 90 95
   Leu Ser Val Phe Gly Gly Gly Thr Lys Leu Thr Val Leu Gly
   100 105 110
<210> 18
   <211> 108
   <212> PRT
   <213> Homo sapiens
<400> 18
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Ser Ser Gly Asn His
   85 90 95
   Val Phe Gly Gly Gly Thr Lys Leu Thr Val Leu Gly
   100 105
<210> 19
   <211> 114
   <212> PRT
   <213> Homo sapiens
<400> 19
   Asp Val Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Pro Pro Phe Thr Phe Gly Gln Gly Thr Lys Leu Glu Ile
   100 105 110
   Lys Arg
<210> 20
   <211> 112
   <212> PRT
   <213> Homo sapiens
<400> 20
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Pro Thr Phe Gly Gln Gly Thr Lys Leu Glu Ile Lys Arg
   100 105 110
<210> 21
   <211> 112
   <212> PRT
   <213> Homo sapiens
<400> 21
   Glu Ile Val Met Thr Gln Thr Pro Leu Ser Leu Ser Ile Thr Pro Gly
   1 5 10 15
   Glu Gln Ala Ser Met Ser Cys Arg Ser Ser Gln Ser Leu Leu His Ser
   20 25 30
   Asp Gly Tyr Thr Tyr Leu Tyr Trp Phe Leu Gln Lys Ala Arg Pro Val
   35 40 45
   Ser Thr Leu Leu Ile Cys Glu Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Asp
   85 90 95
   Ala Gln Asp Pro Thr Phe Gly Gln Gly Thr Lys Leu Glu Ile Lys Arg
   100 105 110
<210> 22
   <211> 238
   <212> PRT
   <213> Homo sapiens
<400> 22
   Glu Val Gln Leu Val Glu Ser Gly Gly Gly Val Val Arg Pro Gly Gly
   1 5 10 15
   Ser Leu Arg Leu Ser Cys Ala Ala Ser Gly Phe Thr Phe Asp Asp Tyr
   20 25 30
   Gly Met Ser Trp Val Arg Gln Ala Pro Gly Lys Gly Leu Glu Trp Val
   35 40 45
   Ser Gly Ile Asn Trp Asn Gly Gly Ser Thr Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Leu Lys Met Pro His Ala Trp Gly Gln Gly Thr Leu Val Thr
   100 105 110
   Val Ser Ser Gly Gly Gly Gly Ser Gly Gly Gly Gly Ser Gly Gly Ser
   115 120 125
   Ala Leu Ser Ser Glu Leu Thr Gln Asp Pro Ala Val Ser Val Ala Leu
   130 135 140
   Gly Gln Thr Val Arg Ile Thr Cys Gln Gly Asp Ser Leu Arg Ser Tyr
   145 150 155 160
   Tyr Ala Ser Trp Tyr Gln Gln Lys Pro Gly Gln Ala Pro Val Leu Val
   165 170 175
   Ile Tyr Gly Lys Asn Asn Arg Pro Ser Gly Ile Pro Asp Arg Phe Ser
   180 185 190
   Gly Ser Ser Ser Gly Asn Thr Ala Ser Leu Thr Ile Thr Gly Ala Gln
   195 200 205
   Ala Glu Asp Glu Ala Asp Tyr Tyr Cys Asn Ser Arg Asp Ser Ser Gly
   210 215 220
   Asn His Val Phe Gly Gly Gly Thr Lys Leu Thr Val Leu Gly
   225 230 235
<210> 23
   <211> 242
   <212> PRT
   <213> Homo sapiens
<400> 23
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Asn Ala
   20 25 30
   Trp Met Ser Trp Val Arg Gln Ala Pro Gly Lys Gly Leu Glu Trp Val
   35 40 45
   Gly Arg Ile Lys Ser Lys Thr Asp Gly Gly Thr Thr Asp Tyr Ala Ala
   50 55 60
   Pro Val Lys Gly Arg Phe Thr Ile Ser Arg Asp Asp Ser Lys Asn Thr
   65 70 75 80
   Leu Tyr Leu Gln Met Asn Ser Leu Lys Thr Glu Asp Thr Ala Val Tyr
   85 90 95
   Tyr Cys Ala Arg Asn Pro Lys Leu Val Lys Trp Gly Gln Gly Thr Leu
   100 105 110
   Val Thr Val Ser Ser Gly Gly Gly Gly Ser Gly Gly Gly Gly Ser Gly
   115 120 125
   Gly Ser Ala Leu Gln Ser Val Leu Thr Gln Pro Pro Ser Ala Ser Gly
   130 135 140
   Thr Pro Gly Gln Arg Val Thr Ile Ser Cys Ser Gly Ser Ser Ser Asn
   145 150 155 160
   Ile Gly Ser Asn Tyr Val Tyr Trp Tyr Gln Gln Leu Pro Gly Thr Ala
   165 170 175
   Pro Lys Leu Leu Ile Tyr Arg Asn Asn Gln Arg Pro Ser Gly Val Pro
   180 185 190
   Asp Arg Phe Ser Gly Ser Lys Ser Gly Thr Ser Ala Ser Leu Ala Ile
   195 200 205
   Ser Gly Leu Arg Ser Glu Asp Glu Ala Asp Tyr Tyr Cys Ala Ala Trp
   210 215 220
   Asp Asp Ser Leu Leu Ser Val Phe Gly Gly Gly Thr Lys Leu Thr Val
   225 230 235 240
   Leu Gly
<210> 24
   <211> 238
   <212> PRT
   <213> Homo sapiens
<400> 24
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Trp Lys Ser Leu Leu Met Leu Trp Gly Gln Gly Thr Leu Val Thr
               100                 105                 110
   Val Ser Ser Gly Gly Gly Gly Ser Gly Gly Gly Gly Ser Gly Gly Ser
           115                 120                 125
   Ala Leu Ser Ser Glu Leu Thr Gln Asp Pro Ala Val Ser Val Ala Leu
       130                 135                 140
   Gly Gln Thr Val Arg Ile Thr Cys Gln Gly Asp Ser Leu Arg Ser Tyr
   145                 150                 155                 160
   Tyr Ala Ser Trp Tyr Gln Gln Lys Pro Gly Gln Ala Pro Val Leu Val
                   165                 170                 175
   Ile Tyr Gly Lys Asn Asn Arg Pro Ser Gly Ile Pro Asp Arg Phe Ser
               180                 185                 190
   Gly Ser Ser Ser Gly Asn Thr Ala Ser Leu Thr Ile Thr Gly Ala Gln
           195                 200                 205
   Ala Glu Asp Glu Ala Asp Tyr Tyr Cys Asn Ser Arg Asp Ser Ser Gly
       210                 215                 220
   Asn His Val Phe Gly Gly Gly Thr Lys Leu Thr Val Leu Gly
   225                 230                 235
<310> 25
   <211> 131
   <212> PRT
   <213> Homo sapiens
<400> 25
<210> 26
   <211> 129
   <212> PRT
   <213> Homo sapiens
<210> 27
   <211> 98
   <212> PRT
   <213> Homo sapiens
<400> 27
   Glu Val Gln Leu Val Glu Ser Gly Gly Gly Val Val Arg Pro Gly Gly
   1 5 10 15
   Ser Leu Arg Leu Ser Cys Ala Ala Ser Gly Phe Thr Phe Asp Asp Tyr
   20 25 30
   Gly Met Ser Trp Val Arg Gln Ala Pro Gly Lys Gly Leu Glu Trp Val
   35 40 45
   Ser Gly Ile Asn Trp Asn Gly Gly Ser Thr Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg
<210> 28
   <211> 98
   <212> PRT
   <213> Homo sapiens
<400> 28
   Glu Val Gln Leu Val Glu Ser Gly Gly Gly Val Val Arg Pro Gly Gly
   1 5 10 15
   Ser Leu Arg Leu Ser Cys Ala Ala Ser Gly Phe Thr Phe Asp Asp Tyr
   20 25 30
   Gly Met Ser Trp Val Arg Gln Ala Pro Gly Lys Gly Leu Glu Trp Val
   35 40 45
   Ser Gly Ile Asn Trp Asn Gly Gly Ser Thr Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Leu Tyr His Cys
   85 90 95
   Ala Arg
<210> 29
   <211> 5
   <212> PRT
   <213> Homo sapiens
<400> 29
   Asp Tyr Gly Met Ser
   1 5
<210> 30
   <211> 17
   <212> PRT
   <213> Homo sapiens
<400> 30
   Gly Ile Asn Trp Asn Gly Gly Ser Thr Gly Tyr Ala Asp Ser Val Lys
   1 5 10 15
   Gly
<210> 31
   <211> 100
   <212> PRT
   <213> Homo sapiens
<400> 31
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Asn Ala
   20 25 30
   Trp Met Ser Trp Val Arg Gln Ala Pro Gly Lys Gly Leu Glu Trp Val
   35 40 45
   Gly Arg Ile Lys Ser Lys Thr Asp Gly Gly Thr Thr Asp Tyr Ala Ala
   50 55 60
   Pro Val Lys Gly Arg Phe Thr Ile Ser Arg Asp Asp Ser Lys Asn Thr
   65 70 75 80
   Leu Tyr Leu Gln Met Asn Ser Leu Lys Thr Glu Asp Thr Ala Val Tyr
   85 90 95
   Tyr Cys Ala Arg
   100
<210> 32
   <211> 100
   <212> PRT
   <213> Homo sapiens
<400> 32
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Asn Ala
   20 25 30
   Trp Met Ser Trp Val Arg Gln Ala Pro Gly Lys Gly Leu Glu Trp Val
   35 40 45
   Gly Arg Ile Lys Ser Lys Thr Asp Gly Gly Thr Thr Asp Tyr Ala Ala
   50 55 60
   Pro Val Lys Gly Arg Phe Thr Ile Ser Arg Asp Asp Ser Lys Asn Thr
   65 70 75 80
   Leu Tyr Leu Gln Met Asn Ser Leu Lys Thr Glu Asp Thr Ala Val Tyr
   85 90 95
   Tyr Cys Thr Thr
   100
<210> 33
   <211> 5
   <212> PRT
   <213> Homo sapiens
<400> 33
   Asn Ala Trp Met Ser
   1 5
<210> 34
   <211> 19
   <212> PRT
   <213> Homo sapiens
<400> 34
   Arg Ile Lys Ser Lys Thr Asp Gly Gly Thr Thr Asp Tyr Ala Ala Pro
   1 5 10 15
   Val Lys Gly
<210> 35
   <211> 98
   <212> PRT
   <213> Homo sapiens
<400> 35
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Trp
<210> 36
   <211> 98
   <212> PRT
   <213> Homo sapiens
<400> 36
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg
<210> 37
   <211> 5
   <212> PRT
   <213> Homo sapiens
<400> 37
   Ser Tyr Ala Met Ser
   1               5
<210> 38
   <211> 17
   <212> PRT
   <213> Homo sapiens
<400> 38
   Ala Ile Ser Gly Ser Gly Gly Ser Thr Tyr Tyr Ala Asp Ser Val Lys
   1               5                   10                  15
   Gly
<210> 39
   <211> 6
   <212> PRT
   <213> Homo sapiens
<400> 39
   Leu Lys Met Pro His Ala
   1 5
<210> 40
   <211> 6
   <212> PRT
   <213> Homo sapiens
<400> 40
   Asn Pro Lys Leu Val Lys
   1 5
<210> 41
   <211> 6
   <212> PRT
   <213> Homo sapiens
<400> 41
   Lys Ser Leu Leu Met Leu
   1 5
<210> 42
   <211> 96
   <212> PRT
   <213> Homo sapiens
<400> 42
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Ser Ser Gly Asn His
   85 90 95
<210> 43
   <211> 11
   <212> PRT
   <213> Homo sapiens
<400> 43
   Gln Gly Asp Ser Leu Arg Ser Tyr Tyr Ala Ser
   1 5 10
<210> 44
   <211> 7
   <212> PRT
   <213> Homo sapiens
<400> 44
   Gly Lys Asn Asn Arg Pro Ser
   1 5
<210> 45
   <211> 9
   <212> PRT
   <213> Homo sapiens
<400> 45
   Asn Ser Arg Asp Ser Ser Gly Asn His
   1 5
<210> 46
   <211> 98
   <212> PRT
   <213> Homo sapiens
<400> 46
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ser Asn
   20 25 30
   Tyr Val Tyr Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Ser Leu
   85 90 95
   Leu Ser
<210> 47
   <211> 98
   <212> PRT
   <213> Homo sapiens
<400> 47
   Gln Ser Val Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Ser Asn
   20 25 30
   Tyr Val Tyr Trp Tyr Gln Gln Leu Pro Gly Thr Ala Pro Lys Leu Leu
   35 40 45
   Ile Tyr Arg Asn Asn Gln Arg Pro Ser Gly Val Pro Asp Arg Phe Ser
   50 55 60
   Gly Ser Lys Ser Gly Thr Ser Ala Ser Leu Ala Ile Ser Gly Leu Arg
   65 70 75 80
   Ser Glu Asp Glu Ala Asp Tyr Tyr Cys Ala Ala Trp Asp Asp Ser Leu
   85 90 95
   Ser Gly
<210> 48
   <211> 13
   <212> PRT
   <213> Homo sapiens
<400> 48
   Ser Gly Ser Ser Ser Asn Ile Gly Ser Asn Tyr Val Tyr
   1 5 10
<210> 49
   <211> 7
   <212> PRT
   <213> Homo sapiens
<400> 49
   Arg Asn Asn Gln Arg Pro Ser
   1 5
<210> 50
   <211> 9
   <212> PRT
   <213> Homo sapiens
<400> 50
   Ala Ala Trp Asp Asp Ser Leu Leu Ser
   1 5
<210> 51
   <211> 96
   <212> PRT
   <213> Homo sapiens
<400> 51
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Ser Ser Gly Asn His
   85 90 95
<210> 52
   <211> 11
   <212> PRT
   <213> Homo sapiens
<400> 52
   Gln Gly Asp Ser Leu Arg Ser Tyr Tyr Ala Ser
   1 5 10
<210> 53
   <211> 7
   <212> PRT
   <213> Homo sapiens
<400> 53
   Gly Lys Asn Asn Arg Pro Ser
   1 5
<210> 54
   <211> 9
   <212> PRT
   <213> Homo sapiens
<400> 54
   Asn Ser Arg Asp Ser Ser Gly Asn His
   1 5
<210> 55
   <211> 16
   <212> PRT
   <213> Homo sapiens
<400> 55
   Arg Ser Ser Gln Ser Leu Leu His Ser Asn Gly Tyr Asn Tyr Leu Asp
   1 5 10 15
<210> 56
   <211> 7
   <212> PRT
   <213> Homo sapiens
<400> 56
   Leu Gly Ser Asn Arg Ala Ser
   1 5
<210> 57
   <211> 9
   <212> PRT
   <213> Homo sapiens
<400> 57
   Met Gln Ala Leu Gln Thr Pro Pro Phe
   1 5
<210> 58
   <211> 7
   <212> PRT
   <213> Homo sapiens
<400> 58
   Met Gln Ala Leu Gln Thr Pro
   1 5
<210> 59
   <211> 102
   <212> PRT
   <213> Homo sapiens
<400> 59
   Asp Val Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Pro Pro Phe
   100
<210> 60
   <211> 100
   <212> PRT
   <213> Homo sapiens
<400> 60
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Pro
   100
<210> 61
   <211> 100
<212> PRT
   <213> Homo sapiens
<400> 61
   Glu Ile Val Met Thr Gln Thr Pro Leu Ser Leu Ser Ile Thr Pro Gly
   1               5                   10                  15
   Glu Gln Ala Ser Met Ser Cys Arg Ser Ser Gln Ser Leu Leu His Ser
               20                  25                  30
   Asp Gly Tyr Thr Tyr Leu Tyr Trp Phe Leu Gln Lys Ala Arg Pro Val
           35                  40                  45
   Ser Thr Leu Leu Ile Cys Glu Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Asp
                   85                  90                  95
   Ala Gln Asp Pro
               100

## Description

### FIELD OF THE INVENTION

The subject invention is directed generally to human platelet glycoprotein Ib alpha, and more particularly to obtaining variable heavy chain and variable light chain regions of antibodies to human platelet glycoprotein Ib alpha and uses thereof.

### BACKGROUND OF THE INVENTION

Throughout this application various publications are referenced, many in parenthesis. Full citations for each of these publications are provided at the end of the Detailed Description.

The platelet glycoprotein Ib/IX (GPIb/IX) receptor for von Willebrand factor (vWf) is believed to consist of a 1:1 heterodimeric complex (Du et al. 1987) between GPIb (160 kDa) and GPIX (17 kDa) in a noncovalent association. GPIb in turn consists of a disulfide-linked 140 kDa alpha chain (GPIb alpha) and a 22 kDa beta chain (GPIb beta) (Fitzgerald and Phillips 1989).

The GPIb/IX complex comprises one of the major transmembrane receptor complexes on blood platelets (Roth 1991; Lopez 1994; Clemetson and Clemetson 1995), mediating von Willebrand factor (vWF)-dependent platelet adhesion. In the 1980's, Miller et al. developed a series of monoclonal antibodies (mab) directed against the GP Ib/IX complex receptor for vWf. In particular, monoclonal antibody C-34 was characterized in detail and it was determined that mab C-34 recognized an epitope within the platelet glycoprotein Ib/IX complex (Miller et al. 1990). In this and subsequent work, Miller et al. showed that monoclonal antibodies C-34, AS-2 and AS-7 were potent inhibitors of the ristocetin-induced aggregation of normal platelets that was dependent upon von Willebrand factor. Miller et al. also showed that the epitopes for all three monoclonal antibodies lay within the GPIb/IX complex.

Attempts to define the binding sites for various monoclonal antibodies have led to the development of epitope libraries. Parmley and Smith developed a bacteriophage expression vector that could display foreign epitopes on its surface (Parmley and Smith 1988). This vector could be used to construct large collections of bacteriophage which could include virtually all possible sequences of a short (e.g. six-amino-acid) peptide. They also developed biopanning, which is a method for affinity-purifying phage displaying foreign epitopes using a specific antibody (see Parmley and Smith 1988; Cwirla et al. 1990; Scott and Smith 1990; Christian et al. 1992; Smith and Scott 1993).

After the development of epitope libraries, Smith et al. then suggested that it should be possible to use the bacteriophage expression vector and biopanning technique of Parmley and Smith to identify epitopes from all possible sequences of a given length. This led to the idea of identifying peptide ligands for antibodies by biopanning epitope libraries, which could then be used in vaccine design, epitope mapping, the identification of genes, and many other applications (Parmley and Smith 1988; Scott 1992).

Antibody fragments have also been displayed on the surface of filamentous phage that encode the antibody genes (Hoogenboom and Winter 1992; McCafferty et al. 1990; Vaughan et al. 1996; Tomlinson et al. 1992; Nissim et al. 1994; Griffiths et al. 1994). Variable heavy chain (V_{H}) and variable light chain (V_{L}) immunoglobulin libraries have thus been developed in phage, and phage can be selected by panning with antibody. The encoded antibody fragments can then be secreted as soluble fragments from infected bacteria. This display of antibodies on phage and selection with antigen mimics immune selection and can be used to make antibodies without immunization from a single library of phage (see Hoogenboom and Winter 1992).

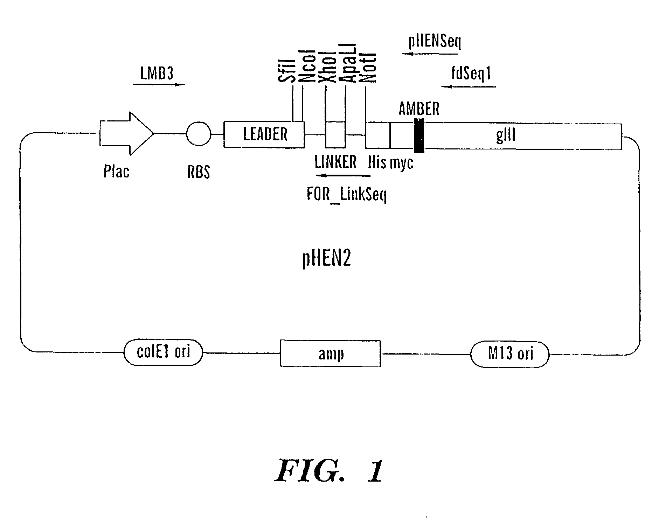

A human synthetic V_{H} and V_{L} ScFv library was made by recloning the heavy and light chain variable regions from the lox library vectors (Griffiths et al. 1994) into the phagemid vector pHEN2 (see Fig. 1). This "Griffin.1" library is a ScFv phagemid library made from synthetic V-gene segments. The World Wide Web address to download the germline V gene sequences which comprise the Griffin.1 library is http://www.mrc-cpe.cam.ac.uk/ imt-doc/vbase-questions.html.

Ware *et al* (1993) *J. Biol. Chem.* **268**, 8376-8382 relates to the expression of human platelet glycoprotein Ibα in transgenic mice.

WO 97/18236 relates to mimotopes and anti-mimotopes of human platelet glycoprotein Ib/IX.

US 5,817,748 relates to mimotopes of human platelet glycoprotein Ib/IX.

Nissim *et al* (1994) *EMBO J.* **13**, 692-698 relates to antibody fragments from a "single pot" phage display library as immunochemical reagents.

Griffiths *et al* (1994) *EMBO J.* **13**, 3245-3260 relates to isolation of high affinity human antibodies directly from large synthetic repertoires.

A need continues to exist for the elucidation of the sequence of useful epitopes of antibodies that bind to glycoprotein Ib alpha.

### SUMMARY OF THE INVENTION

To this end, the subject invention provides a method of selecting a clone that binds to human platelet glycoprotein Ib alpha using a human variable heavy chain and variable light chain immunoglobulin library. The method comprises: incubating a human variable heavy chain and variable light chain immunoglobulin library with cells expressing human platelet glycoprotein Ib, and selecting clones of the library which bind to the cells; and incubating the selected clones of the library with washed human platelets, and selecting resulting clones which bind to the washed human platelets, wherein the resulting clones bind to human platelet glycoprotein Ib alpha.

The subject invention may be used to isolate nucleic acid molecule encoding a variable heavy chain or a variable light chain region of an antibody, wherein the antibody binds to human platelet glycoprotein Ib alpha and inhibits aggregation of platelets.

The isolated nucleic acid molecules selected can be inserted into suitable expression vectors and/or host cells. Expression of the nucleic acid molecules encoding a variable heavy chain or a variable light chain region results in production of variable heavy chain or variable light chain regions of an antibody (wherein the antibody binds to human platelet glycoprotein Ib alpha and inhibits aggregation of platelets) in a host cell.

### BRIEF DESCRIPTION OF THE DRAWINGS

These and other features' and advantages of this invention will be evident from the following detailed description of preferred embodiments when read in conjunction with the accompanying drawings in which:
Fig. 1 is a map of the pHEN2 phagemid vector;
Fig. 2 illustrates the structure of an antibody;
Fig. 3 illustrates the structure of the Fab-fragment of an antibody;
Fig. 4 illustrates the structure of the Fv-fragment of an antibody;
Fig. 5 illustrates the amino acid sequence with alignment for HIb-1;
Fig. 6 illustrates the amino acid sequence with alignment for HIb-2;
Fig. 7 illustrates the amino acid sequence with alignment for HIb-3;
Fig. 8 illustrates the amino acid sequence with alignment for HTb-5;
Fig. 9 illustrates the amino acid sequence with alignment for HIb-6;
Fig. 10 is a direct western blot of HIb-1 human anti-GaIb alpha;
Fig. 11 shows western blocs of HIb-1, HIb-2 and SZ-2 under non-reduced and reduced conditions;
Fig. 12 illustrates inhibition of ristocetin-induced platelet aggregation by clonal phagemid expressing human V_{H} and VL ScFv;
Fig. 13 illustrates inhibition of botrocetin-induced aggregation of formalin-fixed human platelets; and
Fig. 14 illustrates inhibition of antibody by PRP aggregation induced by ristocetin.

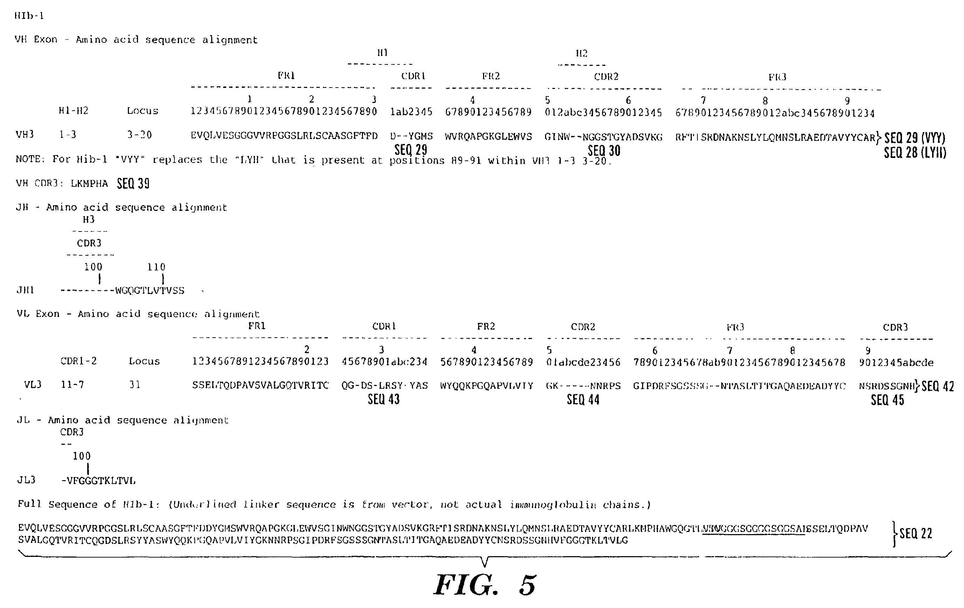

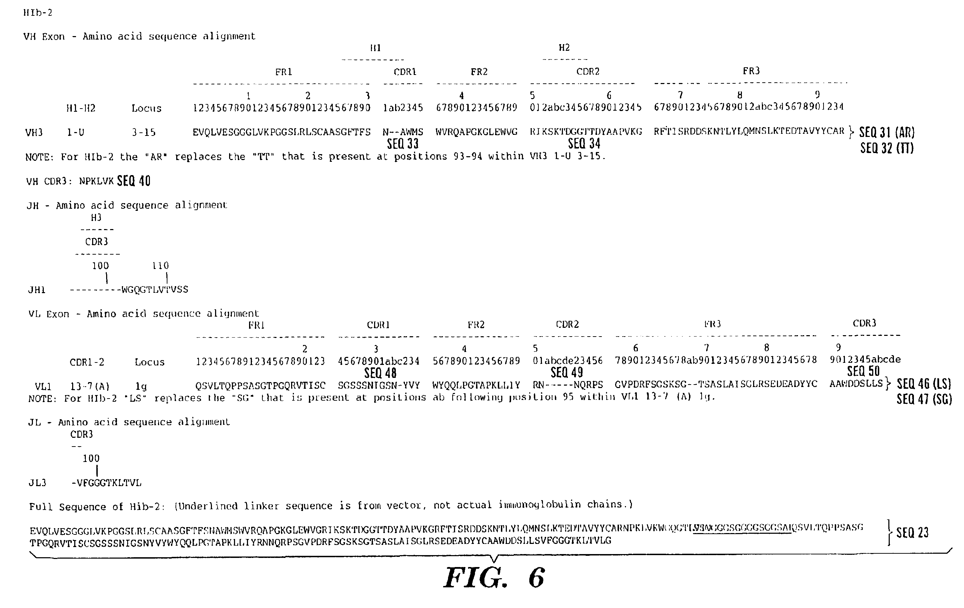

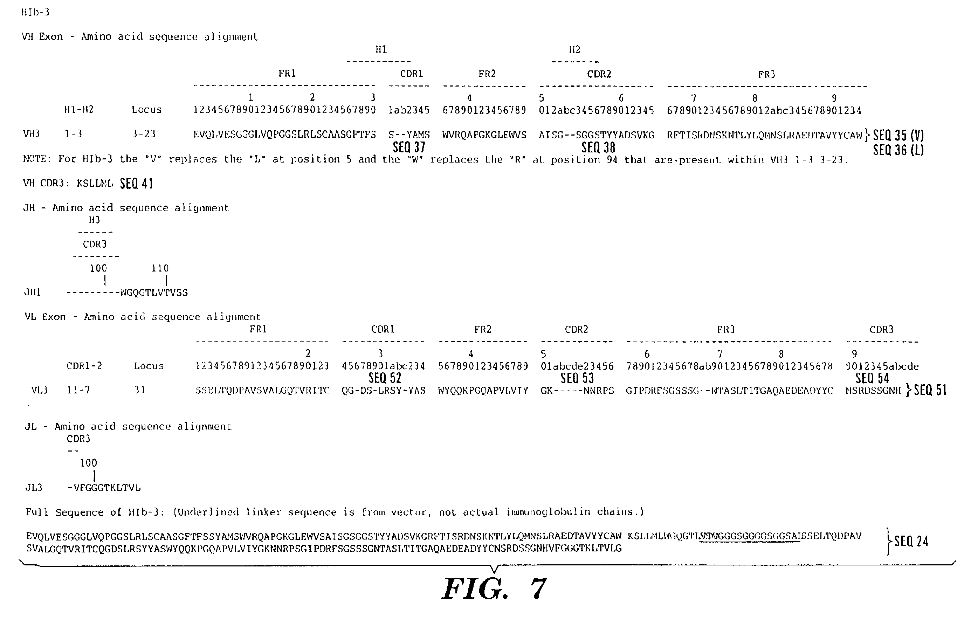

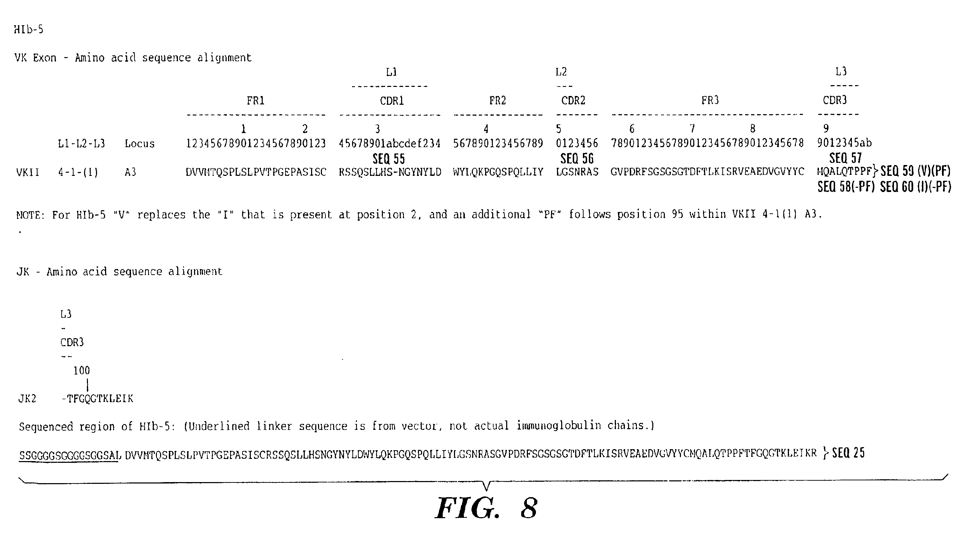

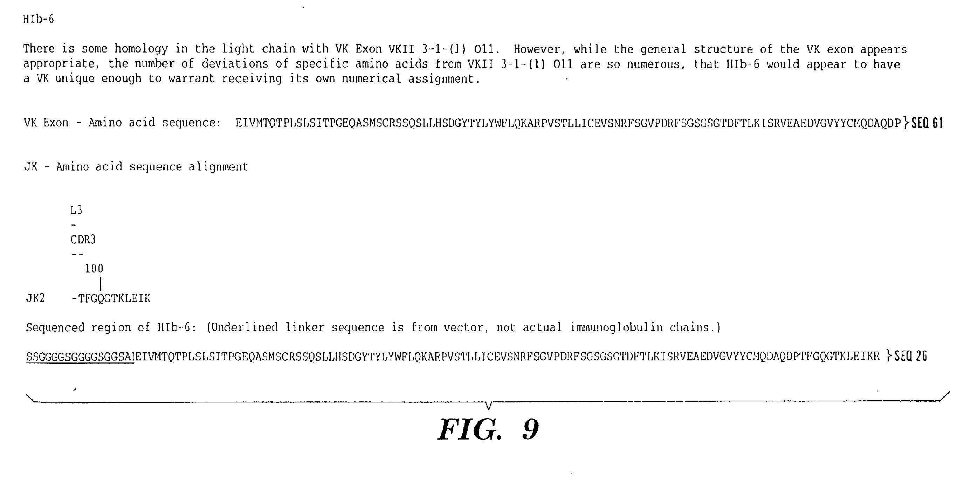

Figures 5 to 14 relate to clones selected by the method of the invention and their use.

### DETAILED DESCRIPTION OF THE INVENTION

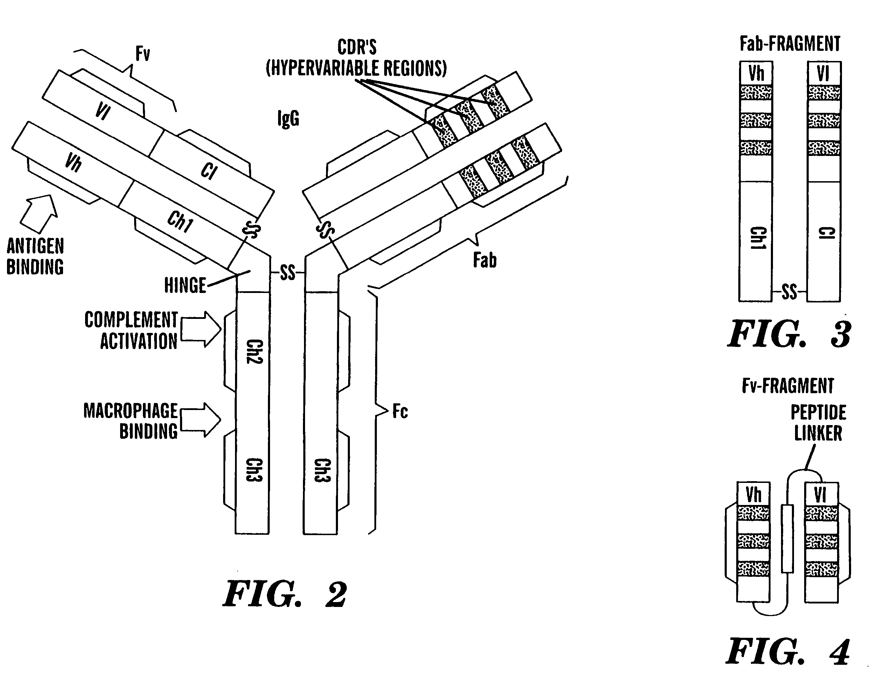

As used herein, antibody, variable heavy chain (V_{H} or. Vh) and variable light chain (V_{L} or Vl) , Pv fragment, and CDR (hypervariable regions) (CDR1 , CDR2, CDR3), are used in the context of Figs. 2-4. Fig. 1 shows a schedmatic drawing of the organization of a natural IgG and derived recombinant fragments. The Fv fragment is shown as a "Single Chain Fv Fragment" (ScFv) in Fig. 4. In this type of recombinant protein, the two antigen binding regions of the light and heavy chain (Vh and V1) are connected by a 15-18 amino acid peptide. This linker region permits appropriate interaction between the Vh and Vl regions. A further description of such recombinant antibody structure can be found at http://www.mgen.uniheidelberg.de/SD/SDscFvSite.html.

The subject invention provides a method of selecting a clone that binds to human platelet glycoprotein Ib alpha using a human variable heavy chain and variable light chain immunoglobulin library. The method comprises: incubating a human variable heavy chain and variable light chain immunoglobulin library with cells expressing human platelet glycoprotein Ib, and selecting clones of the library which bind to the cells; and incubating the selected clones of the library with washed human platelets, and selecting resulting clones which bind to the washed human platelets, wherein the resulting clones bind to human platelet glycoprotein Ib alpha.

Preferably, the cells which express the human platelet glycoprotein Ib alpha are Chinese Hamster Ovary cells.

In one embodiment, the method further comorises incubating the selected resulting clones with further platelets and adding an anti-glycoprotein Ib alpha molecule that may displace clones already bound to the further platelets, and selecting the then-resulting clones that are not bound to the further platelets, the then-resulting clones being capable of binding to human platelet glycoprotein Ib alpha. Preferably, the anti-glycoprotein Ib alpha molecule is a murine monoclonal antibody or peptide (such as the murine monoclonal antibody C-34 or the peptide having the amino acid sequence shown in SEQ ID NO:1).

The method of the subject invention may be used to provide an isolated nucleic acid molecule encoding a variable heavy chain or a variable light chain region of an antibody, wherein the antibody binds to human platelet glycoprotein Ib alpha and inhibits aggregation of platelets. The nucleic acid molecule can be deoxyribonucleic acid (DNA) or ribonucleic acid (RNA, including messenger RNA or mRNA), genomic or recombinant, biologically isolated or synthetic.

The DNA molecule can be a cDNA molecule, which is a DNA copy of a messenger RNA (mRNA) encoding the variable heavy (V_{H}) or variable light (V_{L}) chain.

An example of such a variable heavy chain region of an antibody is the variable heavy chain having a nucleotide sequence selected from the group consisting of SEQ ID NO: 2, SEQ ID NO: 3, and SEQ ID NO: 4. An example of such a variable light chain region of an antibody is the variable light chain having a nucleotide sequence selected from the group consisting of SEQ ID NOs:5-9. The amino acid sequence encoded by these nucleotide sequences are shown in SEQ ID NOs:10-15 (heavy chains), and SEQ ID NOs:16-21 (light chains).

The nucleic acid molecules which may be identified using the method of the subject invention can be expressed in suitable recombinant host cells using conventional techniques. Any suitable host and/or vector syscem can be used to express the variable heavy chain or variable light chain region of an antibody, wherein the antibody binds to human platelet glycoprotein Ib alpha and inhibits aggregation of platelets. For in vitro expression, CHO cells or other mammalian cells, or *Escherichia coli are* preferred.

Techniques for introducing the nucleic acid molecules into the host cells may involve the use of expression vectors which comprise the nucleic acid molecules. These expression vectors (such as phagemids, plasmids, and viruses; viruses including bacteriophage) can then be used to introduce the nucleic acid molecules into suitable host cells. For example, DNA encoding the variable heavy chain or variable light chain region of an antibody can be injected into the nucleus of a host cell or transformed into the host cell using a suitable vector, or mRNA encoding the variable heavy chain or variable light chain region can be injected directly into the host cell, in order to obtain expression of variable heavy chain or variable light chain regions of an antibody in the host cell.

Various methods are known in the art for introducing nucleic acid molecules into host cells. One method is microinjection, in which DNA is injected directly into the nucleus of cells through fine glass needles (or RNA is injected directly into the cytoplasm of cells). Alternatively, DNA can be incubated with an inert carbohydrate polymer (dextran) to which a positively charged chemical group (DEAE, for diethylaminoethyl) has been coupled. The DNA sticks to the DEAE-dextran via its negatively charged phosphate groups. These large DNA-containing particles stick in turn to the surfaces of cells, which are thought to take them in by a process known as endocytosis. Some of the DNA evades destruction in the cytoplasm of the cell and escapes to the nucleus, where it can be transcribed into RNA like any other gene in the cell. In another method, cells efficiently take in DNA in the form of a precipitate with calcium phosphate. In electroporation, cells are placed in a solution containing DNA and subjected to a brief electrical pulse that causes holes to open transiently in their membranes. DNA enters through the holes directly into the cytoplasm, bypassing the endocytotic vesicles through which they pass in the DEAE-dextran and calcium phosphate procedures. DNA can also be incorporated into artificial lipid vesicles, liposomes, which fuse with the cell membrane, delivering their contents directly into the cytoplasm. In an even more direct approach, DNA is absorbed to the surface of tungsten microprojectiles and fired into cells with a device resembling a shotgun.

Several of these methods, microinjection, electroporation, and liposome fusion, have been adapted to introduce proteins into cells. For review, see Mannino and Gould-Fogerite 1988, Shigekawa and Dower 1988, Capecchi 1980, and Klein et al. 1987.

Further methods for introducing nucleic acid molecules into cells involve the use of viral vectors. One such virus widely used for protein production is an insect virus, baculovirus. For a review of baculovirus vectors, see Miller (1989). Various viral vectors have also been used to transform mammalian cells, such as bacteriophage, vaccinia virus, adenovirus, and retrovirus.

As indicated, some of these methods of transforming a cell require the use of an intermediate plasmid vector. U.S. Patent No. 4,237,224 to Cohen and Boyer describes the production of expression systems in the form of recombinant plasmids using restriction enzyme cleavage and ligation with DNA ligase. These recombinant plasmids are then introduced by means of transformation and replicated in unicellular cultures including procaryotic organisms and eucaryotic cells grown in tissue culture. The DNA sequences are cloned into the plasmid vector using standard cloning procedures known in the art, as described by Sambrook et al. (1989).

It should be readily apparent that several of these methods can be used to introduce the nucleic acid molecules into the cells of, or implants of cells within, a subject in vivo (gene therapy applications, including human gene therapy). For example, nucleic acid encoding the variable heavy chain and/or variable light chain, or encoding fragments thereof, or encoding an antibody comprising the variable heavy chain and/or variable light chain or fragments thereof, could be introduced in vivo using a mammalian viral vector such as adenovirus. Such a vector could also include and introduce an inducible promoter controlling expression of the nucleic acid, or other suitable positive or negative response element, so that the subject could simply take a "drug" that would turn on or turn off the expression of the nucleic acid of the subject invention. The "drug", for example, could induce the inducible promoter.

Host cells into which the nucleic acid encoding the variable heavy chain or variable light chain region has been introduced can be used to produce (i.e. to functionally express) the variable heavy chain or variable light chain region. The function of the encoded variable heavy chain or a variable light chain region can be assayed according to methods known in the art by incorporating the variable heavy chain or variable light chain region into an antibody, and testing the antibody for its ability to bind to human platelet glycoprotein Ib alpha and to inhibit aggregation of platelets.

The nucleic acid molecules of the subject invention can be used either as probes or for the design of primers to obtain DNA encoding other variable heavy chain or variable light chain regions of an antibody, wherein the antibody binds to human platelet glycoprotein Ib alpha and inhibits aggregation of platelet, by either cloning and colony/plaque hybridization or amplification using the polymerase chain reaction (PCR).

Specific probes derived from the sequences herein can be employed to identify colonies or plaques containing cloned DNA encoding a variable heavy chain or variable light chain region of an antibody using known methods (see Sambrook et al. 1989). One skilled in the art will recognize that by employing such probes under high stringency conditions (for example, hybridization at 42°C with 5X SSPC and 50% formamide, washing at 50-65°C with 0.5X SSPC), sequences having regions which are greater than 90% homologous or identical to the probe can be obtained. Sequences with lower percent homology or identity to the probe, which also encode variable heavy chain or variable light chain regions of an antibody, can be obtained by lowering the stringency of hybridization and washing (e.g., by reducing the hybridization and wash temperatures or reducing the amount of formamide employed).

Specific primers derived from the sequences herein can be used in PCR to amplify a DNA sequence encoding a variable heavy chain or variable light chain region of an antibody, wherein the antibody binds to human platelet glycoprotein Ib alpha and inhibits aggregation of platelets, using known methods (see Innis et al. 1990). One skilled in the art will recognize that by employing such primers under high stringency conditions (for example, annealing at 50-60°C, depending on the length and specific nucleotide content of the primers employed), sequences having regions greater than 75% homologous or identical to the primers will be amplified.

The invention further provides an antibody comprising the variable heavy chain or variable light chain region disclosed herein. Antibodies of the subject invention include monovalent,' bivalent, and polyvalent antibodies, as well as fragments of these antibodies. Fragments of the antibodies of the present invention include, but are not limited to, the Fab and the F(ab')₂ fragments.

The antibodies which may be obtained using the subject invention may be provided in a detectably labeled form. Antibodies can be detectably labeled through the use of radioisotopes, affinity labels (such as biotin, avidin, etc.), enzymatic labels (such as horseradish peroxidase, alkaline phosphatase, etc.), fluorescent labels (such as FITC or rhodamine, etc.), paramagnetic atoms, etc. Epitope tags can also be used, such as the c-myc peptide (to which antibodies are available that recognize the small peptide or protein of interest). The 6x-histidine tag (for which there are not only antibodies available but also chelating materials that have a high affinity for the histidines) is also commonly used to purify secreted proteins. Procedures for accomplishing such labeling are well known in the art, for example see Sternberger et al. 1970, Bayer et al. 1979, Engval et al. 1972, and Goding 1976.

The antibodies which may be obtained using the method of the invention may be used in a composition comprising the antibody and a carrier. The composition can be used to inhibit aggregation of platelets by exposing platelets to the composition. The antibody can also be used to bind to human platelet glycoprotein Ib alpha, the method comprising exposing human platelet glycoprotein Ib alpha to the antibody.

In the methods of the invention, tissues or cells or platelet glycoprotein Ib alpha (a cell surface protein) are contacted with or exposed to the composition or antibody of the subject invention. In the context of this invention, to "contact" tissues or cells or platelet glycoprotein Ib alpha with or to "expose" tissues or cells or platelet glycoprotein Ib alpha to a composition or antibody means co add the composition or antibody, usually in a liquid carrier, to a cell suspension or tissue sample, either in vitro or ex vivo, or to administer the composition or antibody to cells or tissues within an animal (including humans).

Once a variable heavy chain or variable light chain of interest is identified, the antibody constructed using the variable heavy chain or variable light chain be used to identify peptides capable of mimicking the inhibitory activity of the antibody. One such method utilizes the development of epitope libraries and biopanning of bacteriophage libraries. Briefly, attempts to define the binding sites for various monoclonal antibodies have led to the development of epitope libraries. Parmley and Smith developed a bacteriophage expression vector that could display foreign epitopes on its surface (Parmley, S.F. & Smith, G.P., Gene 73:305-318 (1988)). This vector could be used to construct large collections of bacteriophage which could include virtually all possible sequences of a short (e.g. six-amino-acid) peptide. They also developed biopanning, which is a method for affinity-purifying phage displaying foreign epitopes using a specific antibody (see Parmley, S.F. & Smith, G.P., Gene 73:305-318 (1988); Cwirla, S.E., et al., Proc Natl Acad Sci USA 87:6378-6382 (1990); Scott, J.K. & Smith, G.P., Science 249:386-390 (1990); Christian, R.B., et al., J Mol Biol 227:711-718 (1992); Smith, G.P. & Scott, J.K., Methods in Enzymology 217:228-257 (1993)).

After the development of epitope libraries, Smith et al. then suggested that it should be possible to use the bacteriophage expression vector and biopanning technique of Parmley and Smith to identify epitopes from all possible sequences of a given length. This led to the idea of identifying peptide ligands for antibodies by biopanning epitope libraries, which could then be used in vaccine design, epitope mapping, the identification of genes, and many other applications (Parmley, S.F. & Smith, G.P., Gene 73:305-318 (1988); Scott, J.K., Trends in Biochem Sci 17:241-245 (1992)).

Using epitope libraries and biopanning, researchers searching for epitope sequences found instead peptide sequences which mimicked the epitope, i.e., sequences which did not identify a continuous linear native sequence or necessarily occur at all within a natural protein sequence. These mimicking peptides are called mimotopes. In this manner, mimotopes of various binding sites/proteins have been found.

The sequences of these mimotopes, by definition, do not identify a continuous linear native sequence or necessarily occur in any way in a naturally-occurring molecule, i.e. a naturally occurring protein. The sequences of the mimotopes merely form a peptide which functionally mimics a binding site on a naturally-occurring protein.

Many of these mimotopes are short peptides. The availability of short peptides which can be readily synthesized in large amounts and which can mimic naturally-occurring sequences (i.e. binding sites) offers great potential application.

Using this technique, mimotopes to an antibody that recognizes platelet glycoprotein Ib alpha can be identified. The sequences of these mimotopes represent short peptides which can then be used in various ways, for example as peptide drugs that bind to platelet glycoprotein Ib alpha and inhibit aggregation of platelets. Once the sequence of the mimotope is determined, the peptide drugs can be chemically synthesized.

The antibodies which may be obtained by the method of the subject invention (or fragments thereof) can thus be used to select peptides, mimotopes, etc. that are complementary to the antibodies and that can then be used as antidotes to the antibodies themselves. For example, if a subject being treated with the antibody in order to inhibit platelet aggregation was involved in a motor vehicle accident and the risk of bleeding far exceeded the risk of thrombosis, it would be desirable to turn "off" the antibody of the subject invention. This could be done by using the peptide or mimotope to the antibody itself. The peptide or mimotope could thus be administered to the subject to displace the antibody from the platelets, preventing the antibody-induced inhibition of platelets.

The identified variable heavy chain and variable light chain regions of an antibody, wherein the antibody inhibits aggregation of platelets, can also be used to select additional variable heavy chain and variable light chain regions of an antibody which inhibits aggregation of platelets. Such a method involves the selection of a variable heavy chain or variable light chain region as defined above (for example, SEQ ID NOs:10-15 for heavy chains, SEQ ID NOs:16-21 for light chains), wherein each of the variable heavy chain or variable light chain regions has an amino acid sequence; altering the amino acid sequence of the selected variable heavy chain or variable light chain region; constructing an antibody having the altered amino acid sequence of the variable heavy chain or variable light chain region; and determining whether the antibody inhibits aggregation of platelets, wherein the altered variable heavy chain or variable light chain region of an antibody that inhibits aggregation of platelets is thereby selected.

### EXAMPLE I

Full DNA sequences were isolated from the Human Synthetic VH and VL ScFv Library (the Griffin.1 Library, available from the Medical Research Council in England), and the protein sequences of multiple ScFv clones were determined. The ScFv clones were selected on the basis of their binding to platelet GPIb. Whether displayed as surface proteins on the phagemid or secreted as free ScFv by *E. coli,* several of these different ScFv clones have proven capable of inhibiting von Willebrand factor (vWF)-dependent aggregation of platelets, most likely due to their altering the binding site for vWF that is known to be contained within GPIb. Since the Griffin.1 Library was constructed from native human antibody heavy and light chain variable sequences, ScFv isolated from this library are comprised of native human protein sequences - and hence very attractive potential reagents for therapeutic purposes. The ScFv provide a new class of anti-thrombotic agents, useful for the prevention of platelet-dependent thrombi in diseased arteries, bypass grafts, dialysis access, etc. In contrast to antibodies derived from mouse or other species, the human ScFv stand a far better chance of being recognized as self, rather than as a foreign protein.

In addition to the potential anti-thrombotic uses of the isolated ScFv, these ScFv are also useful as diagnostic reagents in human medicine. Since GPIb is a highly restricted antigen in its expression throughout the body, it has turned out to be one of the best markers to identify platelets, their precursor cell (the megakaryocyte), and leukemic blast cells of megakaryocytic origin. Additionally, there is some evidence in the literature that assaying a soluble form of platelet GPIb in the plasma (that presumably results from proteolytic degradation of platelet surface GPIb) may be useful as a clinical marker. The new anti-GPIb ScFv are readily harvested from E.coli cultures, rather than from the mammalian cells required for murine monoclonal antibodies, and may therefore be a more economical source of anti-GPIb markers for diagnostic uses than were previously available.

Since the human ScFv are directed against platelet glycoprotein Ib, they have been named HIb-1, HIb-2, HIb-3, etc., so as to reinforce the source of the ScFv (Human) and the target of the ScFv (Ib). In the case of HIb-1, HIb-2, and HIb-3, DNA sequencing has provided the amino acid sequences of both the heavy chain and light chain variable regions contributing to the ScFv, including VH exon, particular VH CDR3, JH, linker sequence, VL exon, particular VL CDR3, and JL segments. In the case of HIb-5 and HIb-6, DNA sequencing has thus far provided the amino acid sequences of the light chain variable regions contributing to the ScFv.

This technology provides advantages over existing technology, including:
1. For therapeutic purposes, an anti-platelet antibody of human sequence may obviate the human anti-mouse antibody reaction seen when murine antibodies are used. Platelet GPIb is an important target for anti-thrombotics that is only now beginning to be appreciated.
2. The ScFv are produced by bacterial cultures, which potentially may be more economical than the mammalian cell cultures required for murine antibodies.
3. Since the ScFv are fully cloned, the opportunity to make modifications of the basic ScFv molecules is readily available.
4. Since these ScFv were selected without immunization of animals, it is possible that one or more of these ScFv is directed against an epitope within GPIb for which animals might fail to mount an immune response, due to a high degree of structural conservation across species lines. The Griffin.1 library was constructed in such a manner that ScFv directed against normal human antigens are also included in the repertoire.

The ScFv clones were obtained by screening the Griffin.1 Library. The key points in this screening process were that the first steps in the screening procedure utilized CHO cells expressing recombinant GPIb alpha, and then applicant took the subset of the library surviving three rounds of selection against these cells, and then applicant went into a 4th round against normal washed human platelets. Applicant then did two final rounds where applicant attempted to displace ScFv from washed platelets by flooding them with a lot of murine monoclonal antibody (C-34 or SZ-2) or mimotope peptide (AWNWRYREYV) .

The human synthetic V_{H} and V_{L}ScFv library was made by recloning the heavy and light chain variable regions from the lox library vectors (Griffiths et al. 1994) into the phagemid vector pHEN2 (see Fig. 1). This "Griffin.1" library is a ScFv phagemid library made from synthetic V-gene segments. The World Wide Web address to download the germline V gene sequences which comprise the Griffin.1 library is http://www.mrc-cpe.cam.ac.uk/ imt-doc/vbase-questions.html. The kappa and lambda light chain variable regions were PCR amplified from the fdDOG-2loxVk and VL constructs. The PCR fragments were purified and digested with ApaL1 and Not 1. The gel purified fragments were then ligated into the vector pHEN2. Heavy chain variable regions were PCR amplfied from the pUC19-21oxVH vector. The PCR fragments were purified and digested with Sfil and Xhol. The gel purified fragments were then ligated into the vector Vk-pHEN2 or VL-pHEN2.

The isolation of the HIb series of ScFv was performed as follows:
Initial three rounds of phagemid selection against transfected Chinese Hamster Ovary (CHO) cells expressing only the GPIb alpha component of the GPIb/IX/V complex on their surface
   - 10¹²-10¹³ phagemid incubated 1.5 hours at RT with transfected CHO cells adherent to culture flask
   - Unbound phagemid removed by extensive washing
   - Bound phagemid eluted with triethylamine, neutralized with Tris, infected into *E*. *coli* suppressor strain TG1, and amplified (using helper phage) for use in next round
   - Monoclonal phagemid clone *HIb-3* is a representative clone from this stage of selection
Round 4 of selection: Against washed human platelets
   - 10¹² phagemid incubated with a suspension of washed platelets for 1.5 hours at RT
   - Unbound phagemid removed by extensive washing of platelets
   - Bound phagemid eluted with triethylamine, neutralized with Tris, infected into *E. coli,* and amplified for use in next round
   - Monoclonal phagemid clone *HIb-3* is a representative clone from this stage of selection
Rounds 5 and 6 of selection: Displacement of phage bound to platelets (optional)
   - Round 5: 10¹² Phage from round four incubated with 3x10⁹ washed platelets, unbound phage removed by extensive washing, and platelets then divided into three aliquots
   - Incubation of platelets for 90 min at RT with 25 *µ*g/mL anti-GPIb alpha murine mabs C-34 or SZ-2 or 200 *µ*g/mL C-34 mimotope peptide AWNWRYREYV
   - Phagemid recovered from buffer then infected into *E*. *coli,* and amplified for use in next round
   - Round 6: amplified phage from fifth round bound to washed platelets and then challenged with same potential displacer as used in round 5
Production of ScFv from Round 6 Phagemids
   - Phagemid recovered from round 6 infected into *E*. *coli* non-suppressor strain HB2151
   - 24-well monoclonal culture supernatants assayed in Western blots against platelet lysates and for ability to inhibit ristocetin-induced aggregation of washed human platelets
   - Interesting clones scaled up for DNA sequencing and further functional studies on purified ScFv

This work employs ScFv technology in the development of a new family of antibody molecules directed against human platelet GPIbα--molecules that are themselves derived from human immunoglobulin variable sequences. As an alternative to natural IgG antibodies, synthetic monovalent antibodies with increasingly high affinities can be made from the heavy chain variable and light chain variable regions, separated by a linker region. Such synthetic variable antibodies are termed ScFv. The Griffin.1 synthetic ScFv library, composed of human germline VH and VL sequences used for these studies was produced by the laboratory of Dr. Greg Winter of the MRC in Cambridge, UK. As posted on this laboratory group's web page (http://www.mrc-cpe.cam.ac.uk/-phage/), "This library contains exactly the same synthetic human V-genes as the Human Synthetic Fab (4-12) 2lox Library (Griffiths, A.D. et al., (1994). EMBO J. 13, 3245-3260) but is in a single chain Fv (scFv) format instead of an Fab format. The vector is also a phagemid rather than a phage so it is like the Human Synthetic ScFv Library or "Nissim Library" (Nissim, A. et al., (1994). EMBO J. 13, 692-698) but with diversity in the light chains as well as the heavy chains." In addition to the in vitro recombination of heavy and light chains, this library has achieved an estimated total diversity of 1.2 x 10⁹ clones through in vitro randomization at the hypervariable CDR3 regions. The resulting VH and VL coding sequences were then cloned into the pHEN2 phagemid. Depending upon whether the phagemid is infected into a strain of E.coli lacking or possessing a suppressor for the amber codon, one can then obtain either progeny phagemid expressing the ScFv in fusion with a major phage coat protein, or a secreted form of the ScFv. The secreted ScFv also contain a 6x-His tag which can be used in protein purification and a c-myc tag for detection with an antic-myc antibody such as 9E10.

For the present studies, the initial round of phagemid selection was performed against transfected Chinese Hamster Ovary (CHO) cells expressing only the GPIbα component of the GPIb/IX/V complex on their surface. 10¹²-10¹³ phagemid were incubated for 1.5 hours at RT with transfected CHO cells adherent to the culture flask. Unbound phagemid were removed by extensive washing, and bound phagemid were eluted with triethylamine, neutralized with Tris, infected into the E. coli suppressor strain TG1, and then amplified (using helper phage) for use in the next round. This process was then repeated for two additional rounds.

For the 4^{th} round of selection, we performed a "crossover" step, using human platelets. We aimed by this approach to significantly enrich for phagemid recognizing epitopes present only on both the CHO cell and the platelet, thereby increasing the odds of finding ScFv with specificities for GPIbα. Monoclonal phagemid clone HIb-3 is a representative clone from this stage of selection. Whereas the polyclonal collection of all 4^{th} round phagemid did identify GPIba in Western blots, most individual clones tested did not. Moreover, random conversion of Round 4 phagemid clones to soluble ScFv did not yield ScFv that exhibited inhibitory activity in functional assays.

We accordingly proceeded to additional rounds, designed so as to try to direct the selection of epitopes most relevant to the vWF binding function of GPIbα. Towards this end, phage from round 4 were incubated with washed platelets, and unbound phage removed by extensive washing. Platelets were then further incubated with saturating concentrations of the anti-GPIbα murine mabs C-34 or SZ-2 or with C-34 mimotope peptide, which we have previously shown to compete with platelets for binding to C-34.. Phage recovered in the buffer following these incubations were amplified, and then used in a 6^{th} and final round, in which displacement of phage was again attempted with the same mab or peptide used with it in the previous round.

Phagemid recovered from round 6 were directly infected into the E. coli non-suppressor strain HB2151. Secreted ScFv from overnight supernatants were assayed in Western blots against platelet lysates and were tested for their ability to inhibit ristocetin-induced aggregation of washed human platelets. Interesting clones were then chosen for further study.

A particularly prominent clone ( HIb-1) was observed whether SZ-2, C-34, or C-34 mimotope peptide was used as displacer. Clone HIb-2 was uniquely seen when SZ-2 was used as displacer. Clone HIb-3, as stated above, was derived from an earlier round of selection. Clones HIb-5 and HIb-6 were recovered in the buffer when C-34 mimotope peptide was used as displacer.

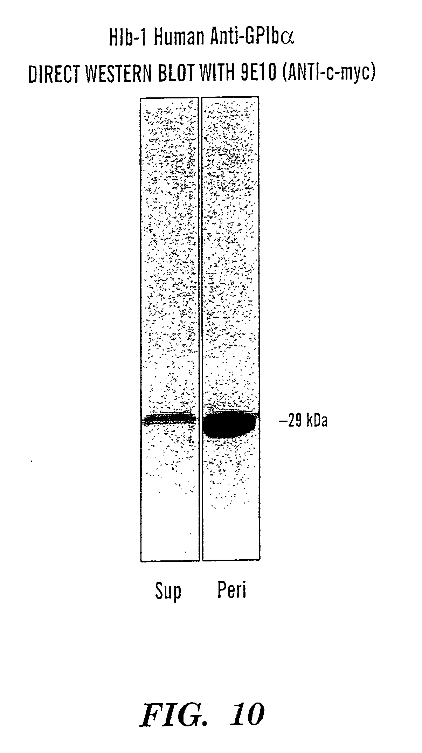

The purified HIb ScFv, whether purified from culture supernatants or from periplasmic spaces, had the anticipated molecular weight of 29 kilodaltons. This is illustrated in Fig. 10, where both crude supernatant and purified periplasmic fraction from an E. coli culture were run on SDS-polyacrylamide gel electrophoresis (SDS-PAGE), electroblotted, and then incubated with the murine monoclonal antibody 9E10, which recognizes the c-myc epitope tag contained within the secreted ScFv. Peroxidase-conjugated secondary anti-mouse antibody was then used to detect the presence of bound 9E10.

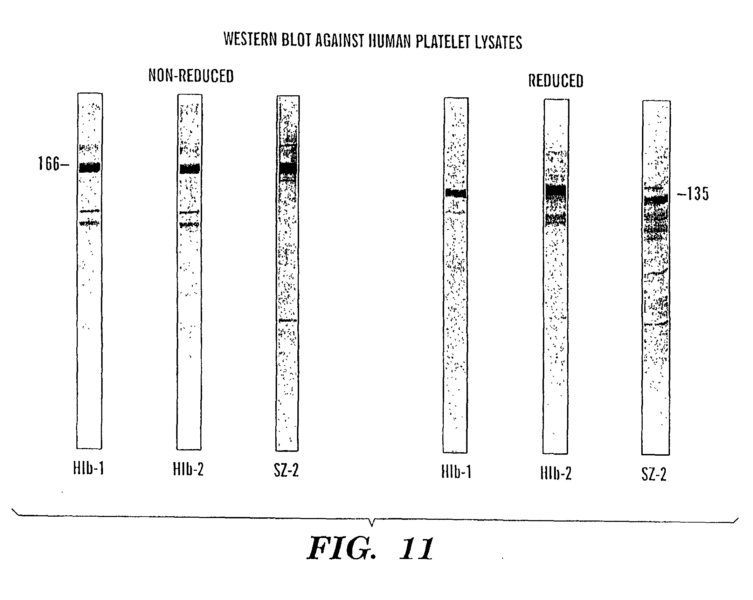

The binding specificity of selected clones with respect to epitope targets deriving from human platelets was also established by immunoblotting. Detergent lysates of platelets obtained from human blood were prepared, and were electrophoresed by SDS-PAGE either under non-reducing conditions or following reduction with b-mercaptoethanol. GPIbα has an apparent molecular mass of 135-140 kDa when electrophoresed under reducing conditions in this system, but characteristically migrates with an apparent molecular mass in the 160-170 kDa region under non-reducing conditions, reflecting the additional mass of GPIbB with which it is covalently bonded in the non-reduced, native state. Following electroblotting to a membrane, the electrophoresed platelet lysates were then probed with either the well-established anti-GPIbα murine monoclonal antibody SZ-2, or with a product from one of the selected clones. Detection of binding of the SZ-2 employed a peroxidase-labeled secondary anti-mouse antibody, as described above. In the earlier rounds of selection, phagemid from selected clones were directly incubated with the blots, and following washings, the residual binding of phagemid was detected through the use of an anti-M13 bacteriophage antibody, since the M13 surface epitope for this antibody is preserved in the pHEN2 phagemid. This approach permitted ready distinction between clones of phagemid that mimicked the binding pattern seen with SZ-2 from those that did not. Following the later rounds of selection, however, when secreted clonal ScFv became available, the ScFv were used instead of actual phagemid in the immunoblotting. Thus, ScFv secreted by clones HIb-1, HIb-2, HIb-5, and HIb-6, when used as the primary antibody in a Western blot against human platelet lysates, all showed a pattern closely mimicking that of murine monoclonal antibody SZ-2. An example of this is shown in Fig. 11, for HIb-1 and HIb-2 ScFv. Following the initial incubation with and subsequent washings of the membranes with the indicated ScFv, secondary antibody 9E10, and in turn peroxidase-conjugated anti-mouse antibody were incubated with the membrane, and staining developed with peroxidase substrate. As can be seen in this example, the products of the selected clones were able to recognize bands having the migration characteristics of GPIbα, both under non-reducing and reducing conditions.

The ability of products from the selected clones to inhibit platelet function was tested by platelet aggregation. Since a major function of GPIbα is its role as receptor for the adhesive ligand, von Willebrand factor (vWF), vWF-dependent platelet aggregation was of particular interest. In vitro, aggregation involving the binding of vWF to platelet GPIb is conventionally assessed using either ristocetin or botrocetin as mediators. ScFv obtained from clones HIbB-1, HIbB-2, HIbB-5, and HIbB-6 were found to inhibit vWF-dependent platelet aggregation induced by at least one of these mediators. In the case of HIB-3, the phagemid itself showed inhibitory activity in an aggregation assay.

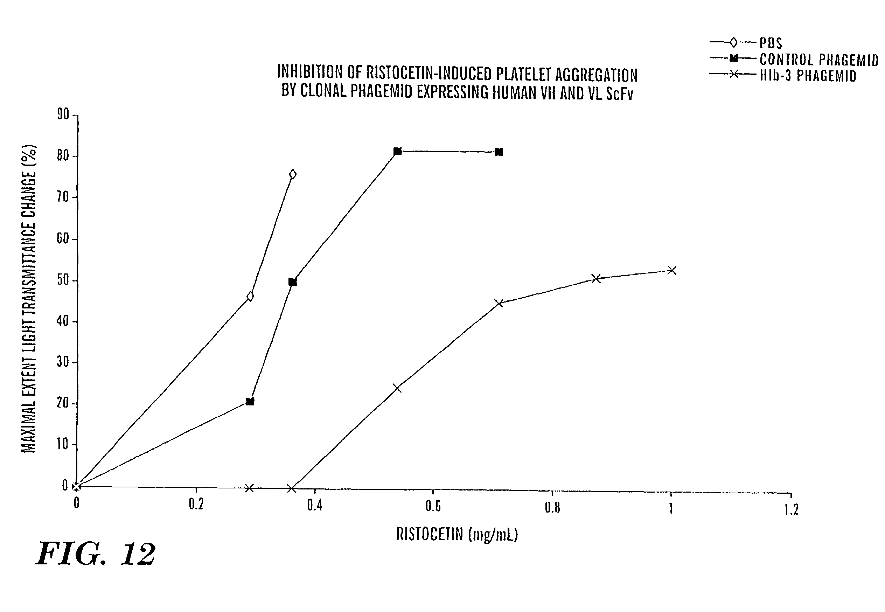

A representative example of inhibition at the phagemid level is shown in Fig. 12. Human platelets that have been formalin-fixed were suspended in buffer containing 5 µg/mL purified vWF at a final platelet concentration of 150,000/µL, added to a cuvette with a stir bar, and stirred at 1200 rpm, 37 °C, in a Chronolog Aggregometer. Ristocetin was then added at varying final concentrations, and resulting change in light transmittance used as an indicator of platelet aggregation. As can be seen in the figure, preincubation for 1.5 hour of the platelets (at 150,000/*µ*L) with 1 x 10¹² HIbB-3 phagemid totally inhibited platelet aggregation at ristocetin concentrations at or below 0.35 mg/mL in this system, and even at a ristocetin concentration as high as 1.0 mg/mL continued to exert strong inhibition. In contrast, in the presence of an equal concentration of phagemid not expressing activity against platelet GPIbα (Control Phagemid), a full aggregatory response was reached by 0.5 mg/mL ristocetin, with moderate aggregation responses observed in the range of 0.3-0.35 mg/mL ristocetin. (Note that the formalin-fixed platelets characteristically are aggregated in the presence of lower concentrations of ristocetin than is usually required with non-fixed platelets.)

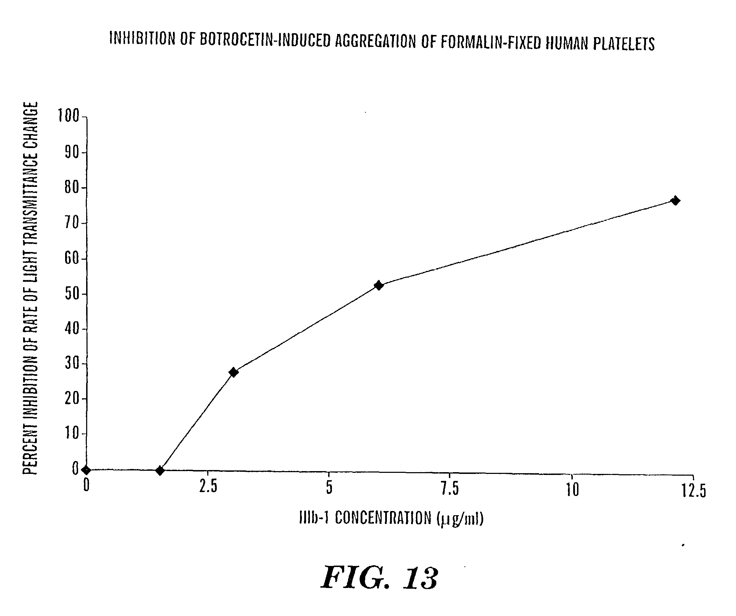

A representative example of inhibition at the ScFv level is shown in Fig. 13. Fixed human platelets were again used under similar conditions to those previously described. In this experiment formalin-fixed human platelets at a concentration of 150,000/*µ*L were incubated for 1.5 hour with HIB ScFv at the indicated final concentration. Purified vWF was than added to a final concentration of 5 µg/mL, the sample put in the aggregometer in the manner described above, and botrocetin added at a final concentration of 0.6 *µ*g/mL to initiate aggregation. In this example, the rate of light transmittance change in the aggregometer is used as an index of aggregation. Quite strong (>75%) inhibition of the aggregation response is observed when the platelets have been preincubated with 12 *µ*g/mL HIB-1. Half-maximal inhibition is characteristically observed in the range of 5-10 *µ*g/mL of HIb-1. Similar results are obtained when ristocetin is used as the agonist. HIB-2. ScFv similarly inhibit the aggregation of human platelets modulated by either ristocetin or botrocetin. HIB-2 ScFv also show half-maximal inhibition of such vWF-dependent platelet aggregation in the range of 5-10 *µ*g/m ScFv, with the maximal degree of such inhibition comparable to or even exceeding that seen with HIB-1. While HIB-5 and HIB-6 also exert inhibition upon vWF-dependent platelet aggregation, the maximal degree of this inhibition has been observed to be weaker than that achieved with HIB-1 or HIB-2, reaching in the range of a 20-30% inhibition of the uninhibited aggregatory response.

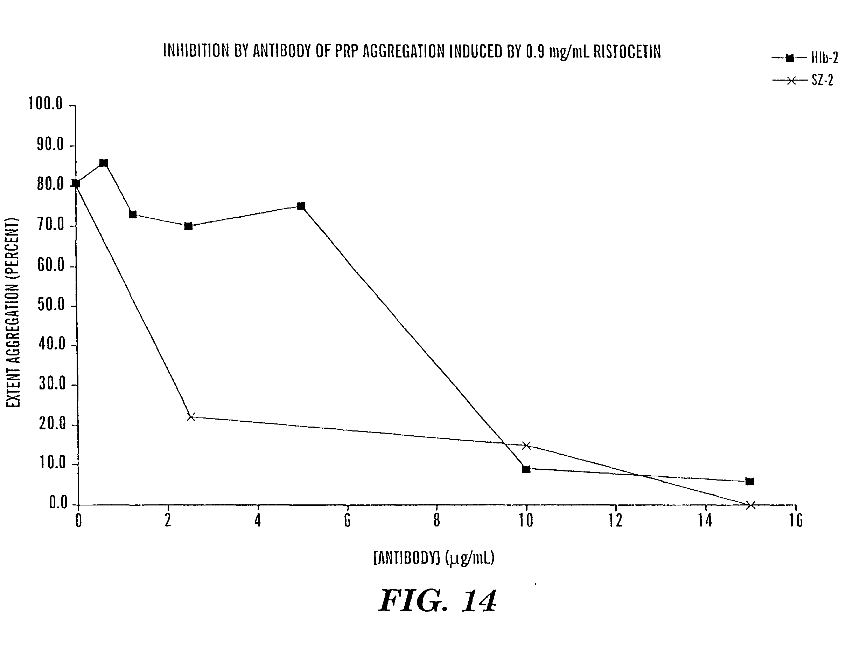

A further representative example, in this instance demonstrating the ability of ScFv purified from the HIB clones to exert inhibitory activity upon unfixed human platelets, is shown in Fig. 14. Here the inhibitory effects of a 1 hour incubation of platelets (150,000 platelets/*µ*L) with either HIB-2 ScFv or with the intact (i.e., full IgG) murine monoclonal antibody SZ-2 are directly compared. In this example, platelet-rich plasma (P.P.) was prepared by centrifugation of citrated, freshly drawn human blood, and the P.P. then studied in the platelet aggregometer under similar conditions as described above. It can be seen that HIB-2 by a concentration of 10 *µ*g/mL was able to produce a degree of aggregation quite comparable to that seen with SZ-2 at the same final concentration.

This work thus demonstrates the discovery of a group of ScFv selected upon the basis of the selection strategy described above, that have been found to inhibit vWF-dependent platelet aggregation induced by botrocetin or ristocetin, and that in fact specifically recognize epitopes within human platelet GPIbα that survive SDS denaturation as well as reduction with mercaptoethanol.

Although preferred embodiments have been depicted and described in detail herein, it will be apparent to those skilled in the relevant art that various modifications, additions, substitutions and the like can be made without departing from the spirit of the invention and these are therefore considered to be within the scope of the invention as defined in the claims which follow.

### REFERENCES

Asfari, M., et al., Endocrinology 130:167-178 (1992).
Bayer, E.A., et al., Meth Enzym 62:308 (1979).
Bhattacharjee, A., et al., Endocrinology 138:3735-3740 (1997).
Boyd, A.E. III, Current Concepts, The Upjohn Company, Kalamazoo, Michigan (1991).
Campbell, A.M., Monoclonal Antibody Technology: Laboratory Techniques in Biochemistry and Molecular Biology, Elsevier Science Publishers, Amsterdam, The Netherlands (1984).
Capecchi, M., Cell 22:479-488 (1980).
Catterall, W.A., Science 242:50-61 (1988).
Catterall, W.A., Science 253:1499-1500 (1991).
Chomczynsk, P., et al., Anal. Biochem. 162:156-157 (1987).
Chrisey, L., et al., Antisense Research and Development 1 (1) :57-63 (1991).
Christoffersen, R.E. and Marr, J.J., Journal of Medicinal Chemistry 38(12):2023-2037 (1995).
Davalli, A.M., et al., J Endocrinology 150:195-203 (1996).
Engval, E., et al., Immunol 109:129 (1972).
Goding, J.W., J Immunol Meth 13:215 (1976).
Han, L., et al., Proc Natl Acad Sci USA 88:4313-4317 (1991).
Hiriart, M. and Matteson, D.R., J Gen Physiol 91:145-159 (1988).
Innis, et al., PCR Protocols, Academic Press, San Diego, CA (1990).
Kato, S., et al., Metabolism 43:1395-1400 (1994).
Kato, S., et al., J Clin Invest 97:2417-2425 (1996).
Keahey, H.H., et al., Diabetes 38:188-193 (1989).
Klein, T.M., et al., Nature 327:70-73 (1987).
Lutz, et al., Exp Cell Res 175:109-124 (1988).
Mannino, R.J. and Gould-Fogerite, S., BioTechniques 6:682-690 (1988).
Miller, L.K., Bioessays 11:91-95 (1989).
Perez-Reyes, E., et al., Nature 391:896-900 (1998).
Rossi, J.J., et al., AIDS Research and Human Retroviruses 8 (2) :183-189 (1992).
Rossi, J.J., British Medical Bulletin 51(1):217-225 (1995).
Sambrook et al., Molecular Cloning: A Laboratory Manual, 2d Edition, Cold Spring Harbor Laboratory Press, Cold Spring Harbor, New York (1989).
Sarver, N., et al., Science 247:1222-1225 (1990).
Seino, S., et al., Proc Natl Acad Sci USA 89:584-588 (1992).
Shigekawa, K. and Dower, W.J., BioTechniques 6:742-751 (1988).
Stea, A., et al., In: Ligand and voltage-gated ion channels. pp113-151, ed. R. Alan North, CRC Press, Boca Raton (1995).
Sternberger, L.A., et al., J Histochem Cytochem 18:315 (1970).
St. Groth, et al., J Immunol Methods 35:1-21 (1980).
Vague, P. and Moulin, J.P., Metabolism 31:139-144 (1982).
Wiltshire, H.R., et al., Xenobiotica 22:837-857 (1992).
Wang, L., et al., Diabetes 45:1678-1683 (1996).
Yaney, G.C., et al., Mol Endocrinol 6:2143-2152 (1992).
Hoogenboom and Winter, J Mol Biol 222:381-388 (1992).
Vaughan et al., Nature Biotech 14:309-315 (1996).
McCafferty et al., Nature 348(6301):552-554 (1990).
Tomlinson et al., J Mol Biol 227:776-798 (1992).
Nissim et al., EMBO J 13(3):692-698 (1994).
Griffiths et al., EMBO J 13(14):3245-3260 (1994).
Hiraiwa et al., Autoimmunity 8:107-113 (1990). Balass, M. et al., Proc Natl Acad Sci USA 90:10638-10642 (November 1993).
Becker, B.H. and Miller, J.L., Blood 74:690-694 (1989).
Chambers, M. et al., in *Leucocyte Typing V: White Cell Differentiation Antigens,* ed. Schlossman, S., pp. 1343-1345, Oxford University Press, New York (1995).
Christian, R.B. et al., J Mol Biol 227:711-718 (1992).
Clemetson, K.J. and Clemetson, J.M., Sem. Thromb. Hemost. 21:130-136 (1995).
Clemetson, K.J. and Hugli, B., in *Leucocyte Typing V*: *White Cell Differentiation Antigens,* ed. Schlossman, S., pp. 1323-1325 Oxford University Press, New York (1995).
Cwirla, S.E. et al., Proc Natl Acad Sci USA 87:6378-6382 (August 1990).
Devlin, J.J. et al., Science 249:404-406 (1990).
Du, X. et al., Blood 69:1524-1527 (1987).
Fitzgerald, L.A. and Phillips, D.R., in Platelet Immunobiology: Molecular and Clinical Aspects, Kunicki, T.J. and George, J.N., Eds., pp. 9-30, Lippincott, Philadelphia PA (1989).
Fox, J.E.B. et al., J. Biol Chem 263:4882-4890 (1988).
Hobart, M.J. et al., Proc R Soc London B 252:157-162 (1993).
Joyce, G.F., Current Opinion in Structural Biology 4:331-336 (1994).
Kupinski, J.M. and Miller, J.L., Thromb Res 43:335-344 (1986).
LaRocca, D. et al., Hybridoma 11:191-201 (1992).
Lenstra, J.A. et al., J Immunol Methods 152:149-157 (1992).
Lopez, J.A., Blood Coag. & Fibrinolysis 5:97-119 (1994).
Luzzago, A. et al., Gene 128:51-57 (1993).
Macfarlane, D.E., et al. Thrombos Diath Haemorrh 34:306-308 (1975).
Miller, J.L. and Castella, A., Blood 60:790-794 (1982).
Miller, J.L. et al., J Clin Invest 72:1532-1542 (1983).
Miller, J.L. et al., Blood 68:743-751 (1986).
Miller, J.L. et al., Blood 70:1804-1809 (1987).
Miller, J.L. et al., Br J Haemotol 74:313-319 (1990).
Miller, J.L. et al., Proc Natl Acad Sci USA 88:4761-4765 (1991).
Miller, J.L. et al., Blood 79:439-446 (1992).
Molino, M. et al., Blood 82:2442-2451 (1993).
Motti, C. et al., Gene 146:191-198 (1994).
Murata, M., et al., J Clin Invest 92:1555-1558 (1993).
Parmley, S.F. and Smith, G.P., Gene 73:305-318 (1988).
Pearson, W.R. and Lipman, D.J., Proc Natl Acad Sci USA 85:2444-2448 (1988).
Pearson, W.R., Methods in Enzymology 183:63-98 (1990).
Roth, G.J., Blood 77:5-19 (1991).
Ruan, C. et al., Blood 69:570-577 (1987).
Russell, S.D. and Roth, G.J., Blood 81:1787-1791 (1993).
Scott, J.K., Trends in Biochem Sci 17:241-245 (1992).
Scott, J.K. and Smith, G.P., Science 249:386-390 (July 27, 1990).
Smith, G.P. and Scott, J.K., Methods in Enzymology 217:228-257 (1993).
Takahashi, H. et al., Thromb Res 19:857-867 (1980).
Takahashi, H. et al., Blood 85:727-733 (1995).
Ward, C.M. and Berndt, M.C., in *Leucocyte Typing V: White Cell Differentiation Antigens,* ed. Schlossman, S., pp. 1336-1337, Oxford University Press, New York (1995).
Weiss, H.J. et al., N Engl J Med 306:326-362 (1982).

## Claims

1. A method of selecting a clone that binds to human platelet glycoprotein Ib alpha using a human variable heavy chain and variable light chain immunoglobulin library, the method comprising:
incubating a human variable heavy chain and variable light chain immunoglobulin library with cells expressing human platelet glycoprotein Ib, and selecting clones of the library which bind to the cells; and
incubating the selected clones of the library with washed human platelets, and selecting resulting clones which bind to the washed human platelets, wherein the resulting clones bind to human platelet glycoprotein Ib alpha.

2. The method of claim 1 wherein the cells are Chinese Hamster Ovary cells.

3. The method of claim 1 further comprising incubating the selected resulting clones with further platelets and adding an anti-glycoprotein Ib alpha molecule that may displace clones already bound to the further platelets, and selecting the then-resulting clones that are not bound to the further platelets, the then-resulting clones being capable of binding to human platelet glycoprotein Ib alpha.

4. The method of claim 3 wherein the anti-glycoprotein Ib molecule is a murine monoclonal antibody.

5. The method of claim 3 wherein the anti-glycoprotein Ib molecule is a peptide.

6. The method of claim 5 wherein the peptide has an amino acid sequence as shown in SEQ ID NO:1 (AWNWRYERYV).

## Patentansprüche

1. Verfahren zum Selektieren eines Klons, der an humanes Thrombozytenglykoprotein Ib alpha anbindet, unter Verwendung einer humanen Immunglobulinbibliothek von variablen Leichtketten und variablen Schwerketten, wobei das Verfahren aufweist:
Inkubieren einer humanen Immunglobulinbibliothek von variablen Leichtketten und variablen Schwerketten mit Zellen, die humanes Thrombozytenglykoprotein Ib expressivieren, und Selektieren von Klonen aus der Bibliothek, die an die Zellen anbinden; und
Inkubieren der selektierten Klone der Bibliothek mit gewaschenen humanen Thrombozyten und Selektieren resultierender Klone, die an die gewaschenen humanen Thrombozyten anbinden, wobei die resultierenden Klone an humanes Thrombozytenglykoprotein Ib alpha anbinden.

2. Verfahren nach Anspruch 1, wobei die Zellen Eierstockzellen des chinesischen Hamsters (CHO-Zellen) sind.

3. Verfahren nach Anspruch 1, das weiterhin aufweist: Inkubieren der selektierten resultierenden Klone mit weiteren Thrombozyten und Hinzufügen eines Antiglykoprotein Ib alpha-Moleküls, das Klone verdrängen kann, die bereits an die weiteren Thrombozyten gebunden sind, und Auswählen der dann resultierenden Klone, die nicht an die weiteren Thrombozyten gebunden sind, wobei die dann resultierenden Klone in der Lage sind, an humanes Thrombozytenglykoprotein Ib alpha anzubinden.

4. Verfahren nach Anspruch 3, wobei das Antiglykoprotein Ib-Molekül ein monoklonaler Mausantikörper ist.

5. Verfahren nach Anspruch 3, wobei das Antiglykoprotein Ib-Molekül ein Peptid ist.

6. Verfahren nach Anspruch 5, wobei das Peptid die Aminosäuresequenz aufweist, die in SEQ ID NO: 1 gezeigt ist (AWNWRYREYV).

## Revendications

1. Procédé pour sélectionner un clone qui se lie à la glycoprotéine plaquettaire humaine Ib alpha, utilisant une banque d'immunoglobulines humaines à chaîne lourde variable et à chaîne légère variable, le procédé comprenant :
l'incubation d'une banque d'immunoglobulines humaines à chaîne lourde variable et à chaîne légère variable avec des cellules exprimant la glycoprotéine plaquettaire humaine Ib, et la sélection de clones de la banque qui se lient aux cellules ; et
l'incubation des clones sélectionnés de la banque avec des plaquettes humaines lavées, et la sélection de clones résultants qui se lient aux plaquettes humaines lavées, où les clones résultants se lient à la glycoprotéine plaquettaire humaine Ib alpha.

2. Procédé selon la revendication 1, dans lequel les cellules sont des cellules d'ovaire de hamster chinois.

3. Procédé selon la revendication 1, comprenant en outre l'incubation des clones résultants sélectionnés avec des plaquettes supplémentaires et l'addition d'une molécule anti-glycoprotéine Ib alpha qui peut déplacer des clones déjà liés aux plaquettes supplémentaires, et la sélection des clones ainsi obtenus qui ne sont pas liés aux plaquettes supplémentaires, les clones ainsi obtenus étant capables de se lier à la glycoprotéine plaquettaire humaine Ib alpha.

4. Procédé selon la revendication 3, dans lequel la molécule anti-glycoprotéine Ib est un anticorps monoclonal murin.

5. Procédé selon la revendication 3, dans lequel la molécule anti-glycoprotéine Ib est un peptide.

6. Procédé selon la revendication 5, dans lequel le peptide a une séquence d'acides aminés telle que représentée par la SEQ ID NO:1 (AWNWRYREYV).
